# EUROPÄISCHE PATENTSCHRIFT

(11) **EP 3 328 909 B1**
(45) Veröffentlichungstag und Bekanntmachung des Hinweises auf die Patenterteilung: **26.06.2019**
(21) Anmeldenummer: 16753261.3
(22) Anmeldetag: 22.07.2016
(51) Int. Cl.: C09D 5/04, C07C 273/18, C07C 275/26, C07C 275/40, C08G 18/67, C08G 18/76, C08G 18/81, C08G 18/32, C09D 7/45

(54) **HARNSTOFFURETHANE ZUR RHEOLOGIESTEUERUNG**
UREA URETHANES FOR RHEOLOGY CONTROL
URÉE-URÉTHANE DESTINÉE À LA COMMANDE RHÉOLOGIQUE

(30) Priorität: 27.07.2015 EP 15178541
(43) Veröffentlichungstag der Anmeldung: 06.06.2018
(73) Patentinhaber: BYK-Chemie GmbH, 46438 Wesel (DE)
(72) Erfinder: EBERHARDT, Marc, 46485 Wesel (DE); NAGELSDIEK, René, 46499 Hamminkeln (DE); BÜHNE, Sylvia, 47198 Duisburg (DE); OMEIS, Jürgen, 46286 Dorsten-Lembeck (DE); KLEIN, Agnetha, 46483 Wesel (DE)
(74) Vertreter: Altana IP Department
(86) Internationale Anmeldenummer: PCT/EP2016/067588
(87) Internationale Veröffentlichungsnummer: WO 2017/017036

(56) Entgegenhaltungen:
- EP-A1- 2 292 675
- US-A1- 2002 115 882

## Beschreibung

Die vorliegende Erfindung betrifft Harnstoffurethane sowie deren Verwendung und diese enthaltende Zubereitungen, deren Herstellung, Verwendung und flüssige Formulierungen, die diese enthalten.

### Hintergrund der Erfindung

Um die Rheologie von flüssigen Systemen, insbesondere flüssigen Beschichtungssystemen oder Bohrspüllösungen, zu steuern, werden als rheologische Hilfstoffe vorwiegend organisch modifizierte Bentonite, Kieselsäuren, hydriertes Rizinusöl und Polyamidwachse eingesetzt.

Nachteilig am Einsatz dieser rheologischen Hilfsstoffe ist, dass diese meist in Form von trockenen Feststoffen vorliegen. Folglich werden deshalb besagte rheologische Hilfsstoffe vor dem Einsatz unter Verwendung von Lösemitteln und Scherkräften zu einem Halbfabrikat aufgeschlossen. Alternativ können die noch nicht aufgeschlossenen rheologischen Hilfsstoffe auch so eingesetzt werden, dass diese durch gezielte Temperatursteuerung in das flüssige System eingebracht werden. Erfolgt diese Temperatursteuerung nicht gemäß den Zielvorgaben, so treten im fertigen Beschichtungssystem typischerweise Kristallite auf, die zu Fehlern in der Beschichtung führen können.

Ein genereller Nachteil des Einsatzes dieser rheologischen Hilfsstoffe ist, dass diese Trübungen und Schleierbildungen (Haze) in klaren, transparenten Beschichtungen verursachen. Außerdem ist der Umgang mit trockenen, pulverförmigen Produkten, die Stäube bei der Verarbeitung verursachen können, unerwünscht.

Eine flüssige Anwendungsalternative zu diesen festen Rheologiesteuerungsmitteln stellen Lösungen von speziellen Harnstoffverbindungen dar.

Derartige Lösungen werden häufig in der Praxis eingesetzt und sind beispielsweise in der Auslegeschrift DE 2822908, der EP-B1 -0006252, der DE-A1 -19919482, der EP-A-1188779 und der EP-A-2370489 beschrieben.

Als Lösemittel beziehungsweise Trägermedium dienen typischerweise polare, aprotische Lösemittel und/oder sogenannte ionische Flüssigkeiten, bei denen es sich de facto um Salzschmelzen handelt, welche bei moderaten Temperaturbedingungen (meist unter 80°C, im Idealfall bei Raumtemperatur) flüssig sind.

Die rheologiesteuernden Eigenschaften gelöster Harnstoffverbindungen sind meist zwar recht gut, allerdings besteht in vielen Fällen Optimierungsbedarf hinsichtlich der Lagerstabilität entsprechender Harnstofflösungen, das heißt die Lösung des Harnstoffs soll keine Niederschläge infolge einer vorzeitigen Harnstoffkristallisation bilden. In der Praxis bedeutet dies, dass eine Lösung des Harnstoffs ohne weiteres gelagert und transportiert werden kann und so ein Einsatz als Rheologiesteuerungsmittel (beispielsweise bei einem Lackhersteller) in einem ausreichenden Zeitfenster nach der eigentlichen Herstellung des Rheologiesteuerungsmittels möglich ist.

Weiterer Optimierungsbedarf besteht hinsichtlich des Wirkungsspektrums als Rheologiesteuerungsmittel, d.h. es besteht Bedarf an Rheologiesteuerungsmitteln, die eine Einsetzbarkeit in Systemen besonders niedriger Polarität ermöglichen, in welchem die bekannten Harnstoffverbindungen nicht mit zufriedenstellendem Ergebnis einsetzbar sind. Somit äußert sich ein optimiertes Verhalten von Rheologiesteuerungsmitteln auf Harnstoffbasis nicht nur in grundsätzlich verbesserter rheologischer Wirksamkeit, sondern in einer Verträglichkeit und gleichzeitig guten Wirksamkeit in besonders unpolaren anwendungsrelevanten Formulierungen (beispielsweise speziellen Bindemittelsystemen, Spüllösungen oder anderen flüssigen Medien), vorzugsweise bei gleichzeitig ausgezeichneter Lagerstabilität der entsprechenden als Rheologiesteuerungsmittel eingesetzten Zusam mensetzungen.

Die Auslegeschrift DE 2822908 beschreibt Thixotropiermittel, die lineare Harnstoffurethane enthalten, welche als Endgruppen Alkylgruppen und/oder alkylterminierte (Poly)alkylenoxidgruppen tragen.

In der EP-B1-0006252 wird ein Verfahren zur Herstellung von Thixotropiermitteln in Gegenwart eines aprotischen Lösemittels und Lithiumchlorid beschrieben. Die erhaltenen Thixotropiermittel besitzen die bereits in der Auslegeschrift DE 2822908 beschriebenen Endgruppen.

Die DE-A1-19919482 offenbart eine Weiterentwicklung des in der EP-B1-0006252 beschriebenen Verfahrens, indem gezielt Monoisocyanataddukte aus Diisocyanaten und Monoalkoholen hergestellt werden, um diese anschließend in Gegenwart von Lithiumsalzen und aprotischen Lösemitteln mit Diaminen umzusetzen. Auch die in der DE-A1-19919482 beschriebenen Harnstoffurethane enthalten ausschließlich gesättigte, gegebenenfalls Heteroatome enthaltende Endgruppen oder Aralkylgruppen als Endgruppen. Ein typischer Vertreter der aus der DE-A1 - 19919482 bekannten Harnstoffurethane wird dort in Beispiel 12 erhalten und basiert auf einem Monoaddukt eines Moleküls n-Dodecanol an ein Molekül Toluylendiisocyanat, welches anschließend mit Xylylendiamin zum Endprodukt umgesetzt wird. Die EP-A1-2370489 stellt eine weitere Fortentwicklung des Verfahrens dar, in welchem auf Lithiumsalze verzichtet werden kann und ionische Flüssigkeiten, das heißt flüssige organische Salze zum Einsatz kommen.

Die EP-A1-1188779 fokussiert auf Harnstoffurethane als Rheologiesteuerungsmittel, die zur Thixotropierung von Formulierungen geeignet sind, die vornehmlich Wasser oder Wasser und geringen Mengen polare organische Lösemittel enthalten. Erfindungswesentlich ist in der EP-A1 -1188779, dass die Endgruppen der Harnstoffurethane zwingend verschieden voneinander sein müssen. In sämtlichen Beispielen der EP-A1-1188779 werden Harnstoffurethane offenbart, bei welchen zumindest eine der beiden Endgruppen eine von Butyltriglykol und/oder Methoxypolyethylenglykol abgeleitete Endgruppe ist. Nur in den Beispielen 8 und 13 werden Harnstoffurethan-Lösungen hergestellt, die neben einer Butyltriglyklol abgeleiteten Endgruppe auch eine unpolare Endgruppe, nämlich eine iso-Tridecylgruppe (abgeleitet vom iso-Tridecanol) besitzen.

Die EP-A1-2292675 wiederum beschreibt polymere Harnstoffe, die über Harnstoff- oder Urethangruppen gebundene gesättigte oder ungesättigte Kohlenwasserstoffgruppen oder eine Vielzahl verschiedener, auch Heteroatome wie Sauerstoff und Stickstoff enthaltende Gruppen als Endgruppen tragen können. Zwar wird in Beispiel 5 auch ein beidseitig oleylterminiertes Produkt beschrieben, welches jedoch unter Verwendung von Isophorondiisocyanat und Hexamethylendiamin erhalten wird.

Die DE 101 27 290 A1 beschreibt thixotrope ungesättigte Polyesterharz-Zusammensetzungen. Die in diesen eingesetzten Thixotropiermittel werden in der Regel in Gegenwart des Polyesterharzes hergestellt. Zur Herstellung werden zunächst Addukte aus aliphatischen Isocyanaten und olefinisch ungesättigten Hydroxyverbindungen hergestellt, die anschließend im Polyesterharz mit aliphatischen oder araliphatischen Aminen umgesetzt werden, ggf. auch unter weiterer Reaktion mit dem Polyesterharz selbst. Die einzigen explizit genannten olefinisch ungesättigten Hydroxyverbindugen sind Hydroxy(meth)acrylsäureester, die später dem Einbau in das Polyesterharz dienen, um dort ihre volle Wirkung zu entfalten. Die Ergebnisse in Bezug auf die Thixotropierwirkung der Thixotropiermittel der DE 101 27 290 A1 deuten jedoch darauf hin, dass der Art des ungesättigten Polyesterharzes eine große Bedeutung zukommt. Ungesättigte langkettige Kohlenwasserstoffreste als Endgruppen von Harnstoffurethanen werden an keiner Stelle der DE 101 27 290 A1 erwähnt.

Somit besteht die Aufgabe der vorliegenden Erfindung insbesondere in der Bereitstellung eines entsprechend qualitativ hochwertigen Rheologiesteuerungsmittels. Dieses soll insbesondere in unpolaren Systemen den bekannten Rheologiesteuerungsmitteln hinsichtlich des Ablaufverhaltens beim Einsatz in Beschichtungsmitteln überlegen sein und /oder auch das Antiabsetzverhalten von Feststoffen in flüssigen Formulierungen verbessern. Ferner sollten die nachteiligen Eigenschaften der aus dem Stand der Technik bekannten Rheologiesteuerungsmittel überwunden werden. Diese und weitere im Folgenden beschriebene Vorzüge wurden durch Bereitstellung der im Folgenden beschriebenen Harnstoffurethane und Harnstoffurethan-Zusammensetzungen erreicht.

### Harnstoffurethane

Die erfindungsgemäße Aufgabe wurde insbesondere gelöst durch Bereitstellung von Harnstoffurethanen der allgemeinen Formel (I) 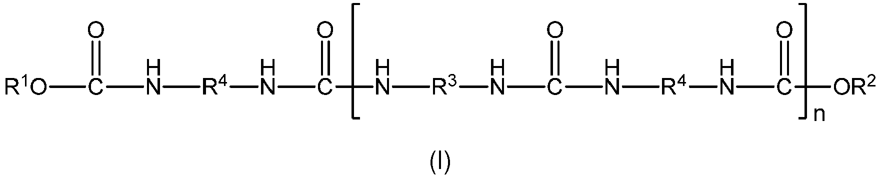 worin
R¹ für einen einfach oder mehrfach ungesättigten, verzweigten oder unverzweigten Alkenylrest mit 12 bis 24 Kohlenstoffatomen, einen einfach oder mehrfach ungesättigten, verzweigten oder unverzweigten Alkinylrest mit 12 bis 24 Kohlenstoffatomen oder für einen mehrfach ungesättigten Kohlenwasserstoff-Rest mit 12 bis 24 Kohlenstoffatomen, der mindestens eine Kohlenstoff-Kohlenstoff-Doppelbindung und mindestens eine Kohlenstoff-Kohlenstoff-Dreifachbindungen aufweist, steht,
R² für einen gesättigten, verzweigten oder unverzweigten Alkylrest mit 8 bis 24 Kohlenstoffatomen oder für einen einfach oder mehrfach ungesättigten, verzweigten oder unverzweigten Alkenylrest mit 12 bis 24 Kohlenstoffatomen, einen einfach oder mehrfach ungesättigten, verzweigten oder unverzweigten Alkinylrest mit 12 bis 24 Kohlenstoffatomen oder für einen mehrfach ungesättigten Kohlenwasserstoff-Rest mit 12 bis 24 Kohlenstoffatomen, der mindestens eine Kohlenstoff-Kohlenstoff-Doppelbindung und mindestens eine Kohlenstoff-Kohlenstoff-Dreifachbindungen aufweist, steht, und
alle n Reste R³ unabhängig voneinander für einen oder mehrere Reste gewählt aus den Struktureinheiten (IIa-o), (IIa-m), (IIa-p), (IIb-o), (IIb-m) und (IIb-p) 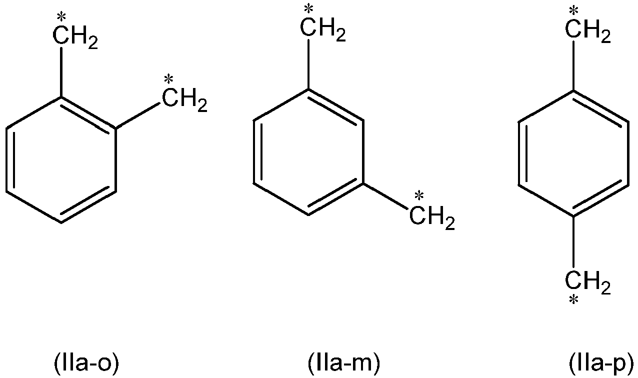 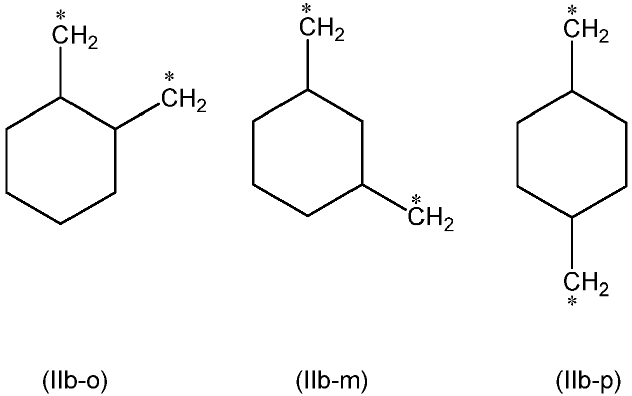
stehen, und
alle n+1 Reste R⁴ unabhängig voneinander für einen oder mehrere Reste gewählt aus den Struktureinheiten (IIIa), (IIIb), (IIIc), (IIId), (IIIe), (IIIf), (IIIg) und (IIIh) 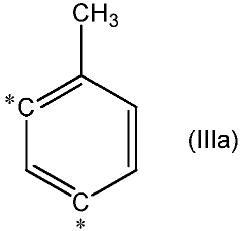 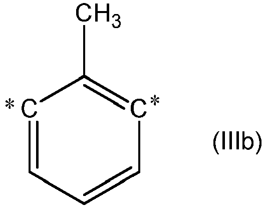 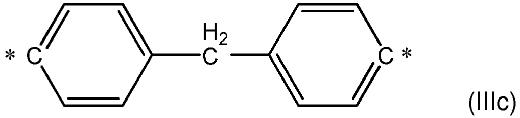 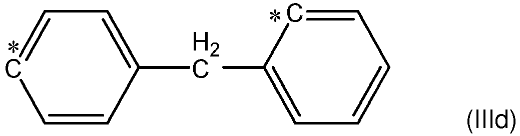 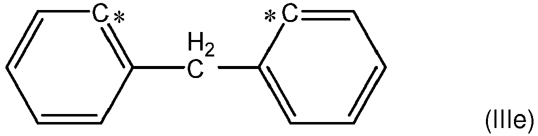 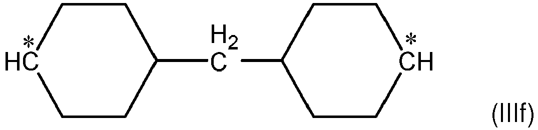 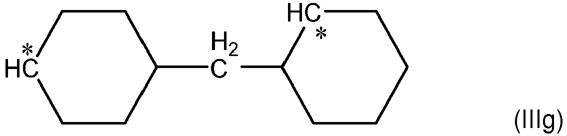 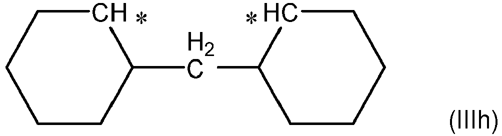
stehen, und
n für eine ganze Zahl ≥ 1 steht, wobei sich die Obergrenze für n aus dem maximalen zahlenmittleren Molekulargewicht Mₙ der Harnstoffurethane der allgemeinen Formel (I) ergibt, welches 65000 g/mol beträgt und welches mittels Gelpermeationschromatographie nach DIN 55672-2 unter Verwendung eines Polymethylmethacrylat-Standards bestimmt wird.

In diesem Sinne ist eine konkrete Angabe eines maximalen Werts für "n" entbehrlich, da dieser durch die feste Obergrenze von Mₙ begrenzt ist. "n" symbolisiert somit lediglich, dass es sich bei den Harnstoffurethanen, um Oligomere oder Polymere handeln kann, wenn n ≠ 1 ist, die n Einheiten [NH-R³-NH-CO-NH-R⁴-NH-CO] enthalten.

Üblicherweise steht n für eine Zahl von 1 bis 150 oder 1 bis 125, vorzugsweise 1 bis 100 oder 1 bis 75 und ganz besonders bevorzugt 1 bis 50 oder 1 bis 25. Weiterhin sind Werte von n = 1 bis 15, 1 bis 10 und 1 bis 5 bevorzugt.

Für n =1 liegen die Harnstoffurethane als Verbindungen mit konkretem Molekulargewicht vor. Diese Verbindungen sind hierin besonders bevorzugt.

Die Harnstoffurethane können jedoch auch oligomer oder polymer sein, das heißt, dass für den Fall n ≥ 2 eine molekulare Uneinheitlichkeit vorliegen kann, in solchen Fällen besitzen diese ein gewichtsmittleres und ein zahlenmittleres Molekulargewicht, wobei diese Mittelwerte in der Regel mit zunehmendem n stärker voneinander abweichen können.

Die zahlenmittleren Molmassen der Harnstoffurethane bis zu etwa 1000 g/mol lassen sich beispielsweise mittels NMR ermitteln, indem die Integrale betreffender NMR-Resonanzsignale ins Verhältnis gesetzt werden. Dem Durchschnittsfachmann ist bekannt, dass für höhere Molekulargewichtsbereiche anstelle der NMR-Spektrokopie zur Bestimmung der Molekulargewichte anderen Verfahren der Vorzug zu geben ist. Die Bestimmung des zahlenmittleren Molekulargewichts der Harnstoffurethane, die eine molare Masse von mehr als 1000 g/mol aufweisen, erfolgt, entsprechend der nachstehenden Beschreibung, als das Zahlenmittel der mittels Gelpermeationschromatographie (GPC) bestimmten Molmassenverteilung. Die GPC-Molmassenverteilung wird gemäß DIN 55672 Teil 2 vom Juni 2008 bestimmt. Als Eluent wird eine Lösung von Lithiumbromid (Gehalt 5 g/l) in Dimethylacetamid verwendet. Zur Kalibrierung werden eng verteilte, linear aufgebaute Polymethylmethacrylat-Standards mit Molekulargewichten zwischen 1000000 und 102 g/mol verwendet. Die Temperatur des Säulensystems beträgt 50°C.

Unter Zugrundelegung der hinsichtlich ihres Molekulargewichts kleinsten Reste R², R³ und R⁴, ergibt sich für n = 1 eine rechnerische Untergrenze des Molekulargewichts der Harnstoffurethane der allgemeinen Formel (I) von 794 g/mol, wenn als R¹ ein dreifach alkenisch ungesättigter Kohlenwasserstoffrest mit 12 Kohlenstoffatomen eingesetzt wird, oder von 792 g/mol, wenn als R¹ ein zweifach alkinisch ungesättigter Kohlenwasserstoffrest eingesetzt wird, oder von 798 g/mol, wenn als R¹ ein einfach alkenisch ungesättigter Kohlenwasserstoffrest eingesetzt wird, beziehungsweise von 796 g/mol, wenn als R¹ ein einfach alkinisch ungesättigter Kohlenwasserstoffrest eingesetzt wird. Da für n = 1 unter vorgenannten Bedingungen einheitliche Verbindungen vorliegen, spricht man üblicherweise vom Molekulargewicht und nicht vom zahlenmittleren Molekulargewicht Mₙ oder gewichtsmittleren Molekulargewicht M_{w}, da bei molekularer Einheitlichkeit das Molekulargewicht = Mₙ = M_{w} ist und es auf die Art der Mittelwertbildung nicht ankommt.

Erfindungsgemäß beträgt die Obergrenze des zahlenmittleren Molekulargewichts Mₙ der erfindungsmäßen Harnstoffurethane der allgemeinen Formel (I) 65000 g/mol, vorzugsweise 50000 g/mol oder 30000 g/mol, besonders bevorzugt 20000 g/mol oder 10000 g/mol und ganz besonders bevorzugt 6000 g/mol, 5000 g/mol oder 4000 g/mol.

Die Bindungsstellen der Reste R³ und R⁴ an die benachbarten NH-Gruppen sind in obigen Formeln durch das Sternsymbol * gekennzeichnet.

Auch wenn es keiner besonderen Definition bedarf, werden hierin die Begriffe Alkylrest, Alkenylrest und Alkinylrest, entsprechend der üblichen chemischen Nomenklatur, als besondere Ausführungsformen aliphatischer Kohlenwasserstoffreste angesehen. Sie stellen somit Reste dar, die entsprechend der IUPAC-Definition eines Kohlenwasserstoffs ausschließlich Kohlenstoff- und Wasserstoffatome enthalten und somit frei von Heteroatomen wie beispielsweise Sauerstoff oder Stickstoff sind.

Die oben dargestellten Harnstoffurethane werden im Folgenden als erfindungsgemäße Harnstoffurethane bezeichnet.

### Harnstoffurethan-Zusammensetzungen

Weiterer Gegenstand der Erfindung sind Harnstoffurethan-Zusammensetzungen enthaltend die erfindungsgemäßen Harnstoffurethane, wobei
der Gewichts-Anteil der Harnstoffurethane der Formel (I), bei welchen beide Reste R¹ und R² ungesättigt sind, 10% bis 100%, bezogen auf die Gesamtheit der Harnstoffurethane der Formel (I), beträgt, und
der Gewichts-Anteil der Harnstoffurethane der Formel (I), bei welchen nur einer der Reste R¹ ungesättigt ist, 0% bis 90%, bezogen auf die Gesamtheit der Harnstoffurethane der Formel (I).

Vorgenannte Harnstoffurethan-Zusammensetzungen werden hierin auch als erfindungsgemäße Harnstoffurethan-Zusammensetzungen bezeichnet.

Die erfindungsgemäßen Harnstoffurethan-Zusammensetzungen können neben den erfindungsgemäßen Harnstoffurethanen der Formel (I) zusätzlich auch von diesen verschiedene Harnstoffurethane enthalten. Solche, von den erfindungsgemäßen Harnstoffurethanen verschiedene Harnstoffurethane, können beispielsweise Harnstoffurethane sein, die Reste R¹, R³ und R⁴ tragen, wie sie erfindungsgemäß definiert sind und einen Rest R² enthalten, der sich von den erfindungsgemäßen Resten R² dadurch unterscheidet, dass er eine geringere oder größere Anzahl an Kohlenstoffatomen enthält und/oder Sauerstoffatome enthält und somit keinen Kohlenwasserstoff-Rest darstellt. Ein derartiger Rest könnte beispielsweise eines oder mehrere Ethersauerstoffatome enthalten, wie dies beispielsweise in einem Di-, Tri- oder Tetraethylenglykolmonobutylether-Rest verwirklicht ist. Sofern derartige nicht-erfindungsgemäße Harnstoffurethane in den erfindungsgemäßen Harnstoffurethan-Zusammmensetzungen enthalten sind, können diese nachträglich in die erfindungsgemäßen Harnstoffurethan-Zusammensetzungen eingebracht werden oder aber in-situ entstehen. Letzteres ist beispielsweise möglich, wenn geringe Mengen der Monoaddukte aus Diisocyanat und nicht erfindungsgemäßem Alkohol bei der Reaktion der erfindungsgemäß einsetzbaren Monoaddukte mit den erfindungsgemäß einsetzbaren Diaminen zugegen sind.

In einer bevorzugten Ausführungsform weisen jedoch mindestens 50 Gew.% aller Harnstoffurethane in der Harnstoffurethan-Zusammensetzung eine Struktur (I) auf. Besonders bevorzugt weisen mindestens 50 Gew.% aller Harnstoffurethane in der Harnstoffurethan-Zusammensetzung eine Struktur (I) auf, in der sowohl R¹ als auch R² ungesättigt, vorzugsweise ethylenisch ungesättigt sind.

In einer weiteren bevorzugten Ausführungsform der erfindungsgemäßen Harnstoffurethan-Zusammensetzungen beträgt der Gewichts-Anteil der Harnstoffurethane der Formel (I), bei welchen beide Reste R¹ und R² ungesättigt sind, 20% bis 100%, bezogen auf die Gesamtheit der Harnstoffurethane der Formel (I), und
der Gewichts-Anteil der Harnstoffurethane der Formel (I), bei welchen nur der Rest R¹ ungesättigt ist, 0% bis 80%, bezogen auf die Gesamtheit der Harnstoffurethane der Formel (I).

In einer bevorzugten Ausführungsform der erfindungsgemäßen Harnstoffurethan-Zusammensetzungen beträgt der Gewichts-Anteil der Harnstoffurethane der Formel (I), bei welchen beide Reste R¹ und R² ungesättigt sind, 50% bis 100%, bezogen auf die Gesamtheit der Harnstoffurethane der Formel (I), und
der Gewichts-Anteil der Harnstoffurethane der Formel (I), bei welchen nur der Rest R¹ ungesättigt ist, 0% bis 50%, bezogen auf die Gesamtheit der Harnstoffurethane der Formel (I).

In einer bevorzugten Ausführungsform der erfindungsgemäßen Harnstoffurethan-Zusammensetzungen beträgt der Gewichts-Anteil der Harnstoffurethane der Formel (I), bei welchen beide Reste R¹ und R² ungesättigt sind, 70% bis 100%, bezogen auf die Gesamtheit der Harnstoffurethane der Formel (I), und
der Gewichts-Anteil der Harnstoffurethane der Formel (I), bei welchen nur der Rest R¹ ungesättigt ist, 0% bis 30%, bezogen auf die Gesamtheit der Harnstoffurethane der Formel (I).

In einer bevorzugten Ausführungsform der erfindungsgemäßen Harnstoffurethan-Zusammensetzungen beträgt der Gewichts-Anteil der Harnstoffurethane der Formel (I), bei welchen beide Reste R¹ und R² ungesättigt sind, 80% bis 100%, bezogen auf die Gesamtheit der Harnstoffurethane der Formel (I), und
der Gewichts-Anteil der Harnstoffurethane der Formel (I), bei welchen nur der Rest R¹ ungesättigt ist, 0% bis 20%, bezogen auf die Gesamtheit der Harnstoffurethane der Formel (I).

In einer bevorzugten Ausführungsform der erfindungsgemäßen Harnstoffurethan-Zusammensetzung beträgt der Gewichts-Anteil der Harnstoffurethane der Formel (I), bei welchen beide Reste R¹ und R² ungesättigt sind, 90% bis 100%, bezogen auf die Gesamtheit der Harnstoffurethane der Formel (I), und
der Gewichts-Anteil der Harnstoffurethane der Formel (I), bei welchen nur der Rest R¹ ungesättigt ist, 0% bis 10%, bezogen auf die Gesamtheit der Harnstoffurethane der Formel (I).

In einer weiteren Ausführungsform enthalten die erfindungsgemäßen Harnstoffurethan-Zusammensetzungen zu 100% Harnstoffurethane der Formel (I), bei welchen beide Reste R¹ und R² ungesättigt, vorzugsweise ethylenisch ungesättigt sind, bezogen auf die Gesamtheit der Harnstoffurethane der Formel (I).

In einer bevorzugten Ausführungsform der erfindungsgemäßen Harnstoffurethane und der diese enthaltenden erfindungsgemäßen Harnstoffurethan-Zusammensetzungen gilt, dass
der Rest R¹ für einen einfach oder mehrfach ungesättigten, verzweigten oder unverzweigten Alkenylrest mit 12 bis 20 Kohlenstoffatomen steht,
der Rest R² für einen gesättigten, verzweigten oder unverzweigten Alkylrest mit 8 bis 20 Kohlenstoffatomen oder einen einfach oder mehrfach ungesättigten, verzweigten oder unverzweigten Alkenylrest mit 12 bis 20 Kohlenstoffatomen steht,
die n Reste R³ unabhängig voneinander für einen oder mehrere Reste gewählt aus den Struktureinheiten (Ila-m) und (Ila-p) stehen, und
die n+1 Reste R⁴ unabhängig voneinander für einen oder mehrere Reste der Struktureinheiten (IIIa) und (IIIb) stehen.

In einer besonders bevorzugten Ausführungsform der erfindungsgemäßen Harnstoffurethane und der diese enthaltenden erfindungsgemäßen Harnstoffurethan-Zusammensetzungen gilt, dass
der Rest R¹ für einen einfach ungesättigten Alkenylrest mit 16 bis 20 Kohlenstoffatomen steht,
der Rest R² für einen gesättigten, verzweigten Alkylrest mit 10 bis 16 Kohlenstoffatomen oder für einen einfach ungesättigten Alkenylrest mit 16 bis 20 Kohlenstoffatomen steht,
die n Reste R³ unabhängig voneinander für einen oder mehrere Reste gewählt aus den Struktureinheiten (IIa-m) und (IIa-p) stehen, und
die n+1 Reste R⁴ unabhängig voneinander für einen oder mehrere Reste der Struktureinheiten (IIIa) und (IIIb) stehen.

In einer ganz besonders bevorzugten Ausführungsform der erfindungsgemäßen Harnstoffurethane und der diese enthaltenden erfindungsgemäßen Harnstoffurethan-Zusammensetzungen gilt, dass
der Rest R¹ für einen unverzweigten Octadecenylrest, vorzugsweise einen Oleylrest steht,
der Rest R² für einen verzweigten oder unverzweigten C₁₀-C₁₄-Rest, vorzugsweise einen Isotridecylrest oder für einen unverzweigten Octadecenylrest, vorzugsweise einen Oleylrest steht,
die n Reste R³ für einen Rest der Struktureinheit (IIa-m) stehen, und die n+1 Reste R⁴ unabhängig voneinander für einen oder mehrere Reste der Struktureinheiten (IIIa) und (IIIb) stehen.

Für alle Ausführungsformen der erfindungsgemäßen Harnstoffurethane und der diese enthaltenden Harnstoffurethan-Zusammensetzungen gilt, dass in den n+1 Resten R⁴ die Struktureinheiten (IIIa) und (IIIb) vorzugsweise in einem molaren Verhältnis von 40 zu 60 bis zu einem molaren Verhältnis von 100 zu 0 stehen, noch bevorzugter, dass in den n+1 Resten R⁴ die Struktureinheiten (IIIa) und (IIIb) in einem molaren Verhältnis von 50 zu 50 bis zu einem molaren Verhältnis von 100 zu 0 stehen und ganz besonders bevorzugt, dass in den n+1 Resten R⁴ die Struktureinheiten (IIIa) und (IIIb) in einem molaren Verhältnis von 60 zu 40 bis zu einem molaren Verhältnis von 100 zu 0 stehen.

Für Ausführungsformen der erfindungsgemäßen Harnstoffurethane und der diese enthaltenden erfindungsgemäßen Harnstoffurethan-Zusammensetzungen gilt, dass vorzugsweise R¹ und R², für einen einfach oder mehrfach ungesättigten, besonders bevorzugt einfach ungesättigten, verzweigten oder unverzweigten, besonders bevorzugt unverzeigten Alkenylrest mit 12 bis 20, besonders bevorzugt 16 bis 20 Kohlenstoffatomen stehen. Besonders bevorzugt gilt für alle Ausführungsformen R¹ = R², ganz besonders bevorzugt gilt R¹ = R² = Oleyl.

Für die praktische Anwendbarkeit ist es zweckmäßig, die erfindungsgemäßen Harnstoffurethan-Zusammensetzungen in flüssiger Form, vorzugsweise in unter Anwendungsbedingungen flüssiger Form, besonders bevorzugt in bei Raumtemperatur, das heißt bei 25°C, flüssiger Form zur Verfügung zu stellen. Vorzugsweise liegen die erfindungsgemäßen Harnstoffurethane in den erfindungsgemäßen Harnstoffurethan-Zusammensetzungen bei Raumtemperatur in gelöster Form vor.

Als Lösemittel geeignet sind insbesondere aprotische organische Lösemittel. Besonders geeignet sind polare, aprotische organische Lösemittel, ganz besonders bevorzugt solche, die gewählt werden aus der Gruppe der Amide, Lactame, Sulfoxide und ionischen Flüssigkeiten (das heißt organische Salze mit einem Schmelzpunkt ≤ 80 °C). Es ist daher bevorzugt, die Herstellung entsprechender erfindungsgemäßer Harnstoffurethan-Zusammensetzungen in diesen polaren, aprotischen organischen Lösemitteln beziehungsweise ionischen Flüssigkeiten durchzuführen.

Besonders bevorzugte polare, aprotische organische Lösemittel sind hierbei substituierte oder unsubstituierte, vorzugsweise unsubstituierte N-Alkylpyrrolidone, Dialkylsulfoxide, substituierte oder unsubstituierte Amide, insbesondere Carbonsäureamide. Beispiele für N-Alkylpyrrolidone sind N-Methylpyrrolidon, N-Ethylpyrrolidon, N-Octylpyrrolidon und N-Hydroxyethylpyrrolidon. Ein Beispiel für ein Dialkylsulfoxid ist Dimethylsulfoxid. Beispiele für Amide sind N,N-Dimethylformamid, N,N-Dimethylacetamid, N,N-Dialkylamidoalkylester, N,N-Dialkylamidoalkylether, Hexamethylphosphorsäuretriamid und Acylmorpholine. Bevorzugte als Lösemittel geeignete ionische Flüssigkeiten sind substituierte Imidazoliumsalze wie z.B. 1-Ethyl-3-methylimidazoliumacetat, 1-Ethyl-3-methylimidazoliumacetat, 1-Ethyl-3-methylimidazoliumethylsulfat, 1-Butyl-3-methylimidazoliumethylsulfat, 1-Ethyl-3-methylimidazoliumthiocyanat und 1-Butyl-3-methylimidazoliumthiocyanat. Entsprechende Lösemittel können auch in Kombinationen eingesetzt werden.

Unter den Lösemitteln sind Dimethylsulfoxid und insbesondere solche N-Alkylpyrrolidone bevorzugt, deren stickstoffgebundener Alkylrest linear oder verzweigt, vorzugsweise linear ist und der Alkylrest 1 bis 20 oder vorzugsweise 2 bis 20, besonders bevorzugt 3 bis 16 und ganz besonders bevorzugt 4 bis 12 Kohlenstoffatome enthält, sowie auch N,N-Dimethylamidoalkylester, N,N-Dimethylamidoalkylether, Formylmorpholin und Acetylmorpholin.

Um die Lösungseigenschaften der Lösemittel zu verbessern, werden häufig auch Salze, das heißt ionogene Verbindungen zugesetzt. Bevorzugt handelt es sich dabei um Salze von Kationen der Hauptgruppen I und II des Periodensystems der Elemente (Alkali- und Erdalkalimetalle) oder um Ammoniumsalze, bevorzugt um Lithium-, Calcium- oder Magnesiumsalze, besonders bevorzugt um Lithium- oder Calciumsalze. Als Anionen bevorzugt sind einwertige Anionen, besonders bevorzugt Halogenid, Pseudohalogenid, Formiat, Acetat und/oder Nitrat, ganz besonders bevorzugt Chlorid, Acetat und/oder Nitrat.

### Herstellung der erfindungsgemäßen Harnstoffurethane sowie der erfindungsgemäßen Harnstoffurethan-Zusammensetzungen

Die Herstellung der erfindungsgemäßen Harnstoffurethan-Zusammensetzung kann in bekannter Weise durch Umsetzung entsprechender Isocyanate mit Aminen erfolgen. Herstellverfahren für solche Harnstoffurethane werden zum Beispiel näher beschrieben in EP-B1-0006252, DE 2822908, DE 19919482, EP 1188779 und EP-A1-2370489. Bevorzugt findet die Herstellung der Harnstoffurethan-Zusammensetzung auch gemäß diesen vorgenannten Herstellverfahren statt.

Bevorzugte Harnstoffurethan-Zusammensetzungen sind daher solche, bei denen die Zusammensetzung gemäß einem solchen Herstellverfahren erhalten wurde.

Beispielsweise können zunächst eines oder mehrere Monoaddukte aus einem oder mehreren Monoalkoholen der Strukturen R¹OH und/oder R²OH und einem oder mehreren Diisocyanaten OCN-R⁴-NCO hergestellt werden, woraus als Addukte Monoaddukte der Formel R¹-O-(CO)-NH-R⁴-NCO beziehungsweise R²-O-(CO)-NH-R⁴-NCO resultiert. Diese Monoaddukte können beispielsweise unter Verwendung eines Überschusses an Diisocyanat erhalten werden, wobei nach deren Herstellung das überschüssige Diisocyanat beispielsweise durch Destillation abgetrennt werden kann.

Die Monoaddukte lassen sich anschließend mit einem oder mehreren Diaminen der Struktur H₂N-R³-NH₂ zum Endprodukt umsetzen.

Anstelle der vorgenannten Diamine können auch ein oder mehrere beidseitig NH₂-terminerte Präpolymere aus einem oder mehreren der vorgenannten Diamine und einem oder mehreren der vorgenannten Diisocyanate hergestellt werden. Anschließend werden das oder die Präpolymere mit dem Monoaddukt umgesetzt.

In einer weiteren beispielhaften Ausführungsform wird das Monoaddukt beziehungsweise werden die Monoaddukte in einer Eintopfreaktion mit einer Mischung aus dem Diamin oder den Diaminen der Struktur H₂N-R³-NH₂ und einem oder mehreren Diisocyanaten OCN-R⁴-NCO zum Harnstoffurethan (I) umgesetzt.

In einer weiteren Alternative wird das Monoaddukt oder werden die Monoaddukte mit einem oder mehreren beidseitig NH₂-terminierten Präpolymeren oder einer Mischung aus einem oder mehreren Diaminen der Struktur H₂N-R³-NH₂ und einem oder mehreren beidseitig NH₂-terminierten Präpolymeren, sowie einem oder mehreren Diisocyanaten der Formel OCN-R⁴-NCO zum Harnstoffurethan (I) umgesetzt.

Es ist auch möglich aus einem Diisocyanat OCN-R⁴-NCO und einem Diamin H₂N-R³-NH₂ ein Präpolymer herzustellen, welches an beiden Enden NCO-terminiert ist und dieses Präpolymer mit Monoalkoholen der Strukturen R¹OH und/oder R²OH umzusetzen, um zu den Harnstoffurethanen der vorliegenden Erfindung zu gelangen.

In allen vorgenannten Fällen ist es jedoch notwendig, dass mindestens ein Monoalkohol der Formel R¹OH zum Einsatz kommt.

Die Umsetzungen finden vorzugsweise in den vorgenannten polaren aprotischen Lösemitteln statt. Harnstoffurethane selbst können, wenn gewünscht, beispielsweise durch destillative Entfernung der Lösemittel erhalten werden.

### Anwendungsbereiche der erfindungsgemäßen Harnstoffurethane sowie der erfindungsgemäßen Harnstoffurethan-Zusammensetzungen

Die erfindungsgemäßen Harnstoffurethane und die diese enthaltenden Harnstoffurethan-Zusammensetzungen zeigen eine besonders gute rheologische Wirksamkeit in unpolaren Bindemittelsystemen wie z.B. Langölalkyden, Mittelölalkyden, NAD-Systemen (non-aqueous dispersions), TPA-Systemen (thermoplastische Acrylate). Die rheologische Wirksamkeit der Zusammensetzung kann beispielsweise anhand des Standvermögens, das heißt der Ablaufgrenze einer entsprechenden Lackformulierung ermittelt werden.

Überraschenderweise können die erfindungsgemäßen Harnstoffurethane beziehungsweise die diese enthaltenden erfindungsgemäßen Harnstoffurethan-Zusammensetzungen auch in an organischen Bindemitteln armen oder sogar von organischen Bindemitteln freien Formulierungen eingesetzt werden, beispielsweise in sogenannten Bohrspüllösungen.

Bei den erfindungsgemäßen Harnstoffurethanen und den diese enthaltenden Harnstoffurethan-Zusammensetzungen handelt es sich daher vorzugsweise um Rheologiesteuerungsmittel, besonders bevorzugt um Thixotropiermittel, die jeweils ebenfalls Gegenstände der vorliegenden Erfindung sind.

Ein weiteres Einsatzgebiet der erfindungsgemäßen Harnstoffurethane und der diese enthaltenden Harnstoffurethan-Zusammensetzungen besteht im Einsatz als Antiabsetzmittel, das heißt als Zusatzstoff, der die Sedimentation von festen Partikeln in flüssigen Systemen verzögert oder gar verhindert; dabei können diese Partikel sowohl anorganischer als auch organischer Natur sein. Als entsprechende Systeme kommen beispielsweise Lack-, Klebstoff-, Schmierstoff- und Kunststoffformulierungen sowie auch Bohrspüllösungen (häufig in Form von W/O-Emulsionen, bei denen die kontinuierliche Phase ein Kohlenwasserstoff und die disperse Phase wässrig ist) sowie nicht-wässrige Slurries (z.B. Slurries von Guar oder Xanthan in einem Kohlenwasserstoff), wie sie bei der Gas- und Ölförderung eingesetzt werden, in Frage.

Die erfindungsgemäßen Harnstoffurethan-Zusammensetzungen zeigen zudem insbesondere in unpolaren Systemen eine ausgeprägte Verträglichkeit, das heißt die Stippen-, Schleier-, und/oder Trübungsbildung in solchen Formulierungen wie beispielsweise Beschichtungsmitteln, insbesondere solchen auf Basis unpolarer Lösemittel, sind gegenüber den aus dem Stand der Technik bekannten Alternativen reduziert beziehungsweise bleiben im Idealfall ganz aus.

Gleichzeitig weisen Lösungen der erfindungsgemäßen Zusammensetzung eine ausgezeichnete Lagerstabilität auf, das heißt es findet in geeigneten Lösungsmitteln, welche auch in Kombination eingesetzt werden können keine Kristallisation der Harnstoffurethane über ausgedehnte Zeiträume statt.

Bevorzugte Lösemittel sind hierbei substituierte oder unsubstituierte, vorzugsweise unsubstituierte N-Alkylpyrrolidone, Dialkylsulfoxide, substituierte oder unsubstituierte Amide, insbesondere Carbonsäureamide. Beispiele für N-Alkylpyrrolidone sind N-Methylpyrrolidon, N-Ethylpyrrolidon, N-Octylpyrrolidon und N-Hydroxyethylpyrrolidon. Ein Beispiel für ein Dialkylsulfoxid ist Dimethylsulfoxid. Beispiele für Amide sind N,N-Dimethylformamid, N,N-Dimethylacetamid, N,N-Dialkylamidoalkylester, N,N-Dialkylamidoalkylether, Hexamethylphosphorsäuretriamid und Acylmorpholine. Bevorzugte als Lösemittel geeignete ionische Flüssigkeiten sind substituierte Imidazoliumsalze wie z.B. 1-Ethyl-3-methylimidazoliumacetat, 1-Ethyl-3-methylimidazoliumacetat, 1-Ethyl-3-methylimidazoliumethylsulfat, 1-Butyl-3-methylimidazoliumethylsulfat, 1-Ethyl-3-methylimidazoliumthiocyanat und 1-Butyl-3-methylimidazoliumthiocyanat.

Unter den Lösemitteln sind Dimethylsulfoxid und insbesondere solche N-Alkylpyrrolidone bevorzugt, deren stickstoffgebundener Alkylrest linear oder verzweigt, vorzugsweise linear ist und vorzugsweise 1 bis 20, besonders bevorzugt 3 bis 16 und ganz besonders bevorzugt 4 bis 12 Kohlenstoffatome enthält, sowie auch N,N-Dimethylamidoalkylester, N,N-Dimethylamidoalkylether, Formylmorpholin und Acetylmorpholin.

Es ist es ist ebenfalls möglich, verschiedene der erfindungsgemäßen Harnstoffurethane, der erfindungsgemäßen Harnstoffurethan-Zusammensetzungen, der erfindungsgemäßen Rheologiesteuerungsmittel, der erfindungsgemäßen Thixotropiermittel oder der erfindungsgemäßen Antiabsetzmittel in Kombination einzusetzen, das heißt eine Mischung solcher Stoffe, Zusammensetzungen oder Mittel, vorzugsweise zur Rheologiesteuerung, insbesondere zur Thixotropierung oder als Antiabsetzmittel zu verwenden.

Weiterer Gegenstand der vorliegenden Erfindung ist daher die Verwendung der erfindungsgemäßen Harnstoffurethane oder der erfindungsgemäßen Harnstoffurethan-Zusammensetzungen als Antiabsetzmittel oder als Rheologiesteuerungsmittel, vorzugsweise als Thixotropiermittel, jeweils in flüssigen, vorzugsweise bei Raumtemperatur (25 °C) flüssigen Formulierungen.

Diese flüssigen Formulierungen sind vorzugsweise Beschichtungsmittel wie beispielsweise Lacke, oder Kunststoffformulierungen, Pigmentpasten, Dichtstoffformulierungen, Kosmetika, Keramikformulierungen, Bohrspüllösungen für Gas- und Ölförderung, nicht-wässrigen Slurries, Reinigungsmittel, Klebstoffformulierungen, Vergußmassen, Baustoffformulierungen, Schmierstoffen, Spachtelmassen, Druckfarben oder Tinten wie beispielsweise Inkjet-Tinten.

Bevorzugte flüssige Formulierungen sind wasserfrei oder enthalten nur geringe Mengen an Wasser. Unter einer geringen Menge an Wasser wird hierin ein Wassergehalt von maximal 5 Gew.-%, vorzugsweise maximal 2 Gew.-% und besonders bevorzugt maximal 1 Gew.-% verstanden.

In einer weiteren besonderen Ausführungsform bildet die flüssige Formulierung die kontinuierliche Phase einer sogenannten Wasser-in-ÖI-Emulsion. Die oben angegebenen Wassermengen beziehen sich dann nur auf die flüssige Formulierung, welche vor der Bildung der Emulsion vorliegt (also die spätere "Ölphase"). Der bei der Bildung der Emulsion von der wässrigen Phase in die kontinuierliche Phase übergehende Anteil an Wasser ist äußerst gering und wird hierin nicht berücksichtigt.

Die Wasser-in-ÖI-Emulsion kann bezogen auf ihr Gesamtgewicht durchaus einen Wassergehalt von beispielsweise 20 oder 30 Gew.-% besitzen. Typische Wasser-in-Öl-Emulsionen sind sogenannte Bohrschlämme, vorzugsweise Ölbohrschlämme, die ebenfalls Gegenstand der vorliegenden Erfindung sind.

Bei den Lacken, Druckfarben und Tinten, insbesondere Inkjettinten kann es sich sowohl um lösemittelhaltige als auch lösemittelfreie Lacke, Druckfarben und Tinten wie Inkjettinten handeln. Lacke sind in unterschiedlichsten Anwendungsfeldern einsetzbar, unter anderem im Bereich Automobillacke, Bautenlacke, Schutzlacke (u.a. Lackierung von Schiffen und Brücken), Can- und Coil-Coating-Lacke, Holz- und Möbellacke, Industrielacke, Kunststofflackierungen, Drahtlacke, Beschichtungen von Lebensmitteln und Saatgut sowie auch als sogenannte Color Resists, welche für Farbfilter beispielsweise in Flüssigkristall-Displays eingesetzt werden. In den Anwendungsbereich der Lacke fallen auch pastöse Materialien, welche in der Regel einen sehr hohen Anteil von Feststoff und einen geringen Anteil von flüssigen Komponenten aufweisen, beispielsweise sogenannte Pigmentpasten oder auch Pasten auf Basis von feinverteilten Metallteilchen oder Metallpulvern (beispielsweise auf Basis von Silber, Kupfer, Zink, Aluminium, Bronze, Messing). Derartige Pasten, enthaltend die erfindungsgemäßen Harnstoffurethane beziehungsweise Harnstoffurethan-Zusammensetzungen sind ebenfalls Gegenstand der vorliegenden Erfindung.

Bei den Kunststoff-Formulierungen handelt es sich um vorzugsweise flüssige, besonders bevorzugt bei Raumtemperatur, das heißt 25 °C, flüssige Formulierungen zur Herstellung von Kunststoff-Materialien, die bevorzugt durch einen chemischen Vernetzungsprozess ("Härtung") zu einem Duromer umgesetzt werden. Bevorzugte Kunststoffzubereitungen sind daher ungesättigte Polyesterharze, Vinylesterharze, Acrylatharze, Epoxidharze, Polyurethanharze, Formaldehydharze (wie MelaminFormaldehyd oder Harnstoff-Formaldehyd). Diese können unter unterschiedlichsten Bedingungen ausgehärtet werden, so beispielsweise bei Raumtemperatur (kalthärtende Systeme) oder bei erhöhter Temperatur (heißhärtende Systeme), gegebenenfalls auch unter Aufbringung von Druck ("Closed Mold"-Applikation, Sheet Molding Compounds oder Bulk Molding Compounds). Zu den bevorzugten Kunststoff-Formulierungen zählen auch PVC-Plastisole.

Bei den Kosmetikzubereitungen kann es sich um mannigfaltige flüssige Zusammensetzungen handeln, die im sogenannten Personal Care- oder auch Health Care-Bereich eingesetzt werden, so zum Beispiel Lotionen, Cremes, Pasten wie beispielsweise Zahnpasta, Schäume wie beispielsweise Rasierschäume, Gele wie beispielsweise Rasiergele, Duschgele, Arzneiwirkstoffe in gelförmigen Formulierungen, Haarshampoos, Flüssigseifen, Nagellacken, Lippenstiften oder Haarfärbemitteln.

Bei den Bohrspüllösungen handelt es sich um Flüssigkeiten, die im Rahmen einer Bohrung durch das Bohrloch gepumpt werden. In der Regel handelt es sich um Suspensionen von festen anorganischen Partikeln, die häufig mittels eines Spezialmischers hergestellt werden. Bevorzugt handelt es sich um sogenannte ölbasierte Bohrspüllösungen, in denen die kontinuierliche flüssige Phase aus einem organischen flüssigen Medium (in der Regel einem Kohlenwasserstoff) besteht, in dem anorganische Bestandteile wie Bariumsulfat suspendiert sind und die häufig auch als inverse Emulsion, das heißt als Emulsion von Wassertröpfchen im organischen Medium, vorliegen. Ebenfalls bevorzugt in der Gas- und Ölförderung eingesetzt werden nicht-wässrige Slurries, bei denen es sich bevorzugt um Suspensionen von organischen Verbindungen, bevorzugt Polysacchariden (z.B. Xanthan oder Guar Gum), in Kohlenwasserstoffen handelt, welche oft als pumpbare Medien mit dem Ziel der späteren Verdickung von wässrigen Medien eingesetzt werden (z.B. beim sog. "hydraulic fracturing").

Bei den Schmierstoffen handelt es sich um Mittel, welche zur Schmierung eingesetzt werden, das heißt die der Verringerung von Reibung und Verschleiß, sowie der Kraftübertragung, Kühlung, Schwingungsdämpfung, Dichtwirkung und dem Korrosionsschutz dienen, wobei hier flüssige Schmierstoffe und Schmierfette bevorzugt sind.

Bei den Klebstoffen kann es sich um alle unter Verarbeitungsbedingungen flüssigen Prozesswerkstoffe handeln, welche Fügeteile durch Flächenhaftung und innere Festigkeit verbinden können. Diese Klebstoffe können bevorzugt lösemittelhaltig oder lösemittelfrei sein.

Schließlich betrifft die vorliegende Erfindung die obengenannten flüssigen Formulierungen, welche die erfindungsgemäßen Harnstoffurethane oder die erfindungsgemäßen Harnstoffurethan-Zusammensetzungen enthalten.

Der Anteil an erfindungsgemäßen Harnstoffurethanen in den erfindungsgemäßen flüssigen Formulierungen, insbesondere den Beschichtungsmitteln, Kunststoffformulierungen, Pigmentpasten, Dichtstoffformulierungen, Kosmetika, Keramikformulierungen, nicht-wässrigen Slurries und Bohrspüllösungen für Gas- und Ölförderung, Klebstoffformulierungen, Vergussmassen, Baustoffformulierungen, Schmierstoffen, Spachtelmassen, Druckfarben oder Tinten beträgt, bezogen auf das Gesamtgewicht der flüssigen Formulierung vorzugsweise 0,1 bis 5 Gew.-%, besonders bevorzugt 0,15 bis 3 Gew.-% und ganz besonders bevorzugt 0,2 bis 2,0 Gew.-%.

Vorzugsweise enthalten die erfindungsgemäßen flüssigen Formulierungen zusätzlich einen oder mehrere Kohlenwasserstoffe ausgewählt aus der Gruppe der aliphatischen, cycloaliphatischen, aromatischen und aralphatischen Kohlenwasserstoffe. Besonders bevorzugt sind der oder die Kohlenwasserstoffe zu mindestens 10 Gew.-% bis 99 Gew.-% bezogen auf das Gesamtgewicht der flüssigen Formulierung in der flüssigen Formulierung enthalten. Bevorzugte Untergrenzen des Gehalts an Kohlenwasserstoffen in der flüssigen Formulierung sind beispielsweise 15 oder 20 Gew.-%. Die bevorzugten Obergrenzen können jedoch auch niedriger als 99 Gew.-% sein, wie beispielsweise 80 Gew.-%, 70 Gew.-% oder gar 25 Gew.-%.

Nachstehend soll die vorliegende Erfindung zusätzlich anhand von Beispielen näher erläutert werden.

### BEISPIELE

Die folgenden Prozentangaben sind, soweit nichts anderes angegeben ist, Gewichtsprozentangaben.

### (I) Synthese der Rheologiesteuerungsmittel

### Vergleichsbeispiele

Vergleichsbeispiel 1 (analog Beispiel 12 der DE-A1-19919482):
Zunächst wird ein Monoaddukt gemäß der Patentschrift EP 1188779 aus 2,4-Toluylendiisocyanat (Desmodur T100, Bayer) und Laurylalkohol hergestellt.

In einem Reaktionsgefäß werden unter Rühren 1,65 g (0,04 mol) LiCl in 75,0 g N-Ethylpyrrolidon (BASF) gelöst. Danach werden 3,55 g (0,026 mol) meta-Xylylendiamin zugegeben und die klare Mischung auf 60°C erwärmt. Anschließend werden 19,8 g (0,052 mol) des Monoaddukts aus Desmodur T100 und Laurylalkohol unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt mit 25% Gehalt an Harnstoffurethan erhalten.

### Vergleichsbeispiel 2:

Zunächst wird ein Monoaddukt gemäß der Patentschrift EP 1188779 aus 2,4-Toluylendiisocyanat (Desmodur T100, Bayer) und Laurylalkohol hergestellt.

In einem Reaktionsgefäß werden unter Rühren 1,65 g (0,04 mol) LiCl in 75,0 g Methyl 5-(dimethylamino)-2-methyl-5-oxopentanoat gelöst. Danach werden 3,55 g (0,026 mol) meta-Xylylendiamin zugegeben und die klare Mischung auf 60°C erwärmt. Anschließend werden 19,8 g (0,052 mol) des Monoaddukts aus Desmodur T100 und Laurylalkohol unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt mit 25% Gehalt an Harnstoffurethan erhalten.

### Vergleichsbeispiel 3:

Als Vergleichsbeispiel 3 wurde das Beispiel 5 aus der EP 2292675 A1 hergestellt.

### Beispiele

Zunächst werden als Bausteine für die weiter unten beschriebenen Synthesen der Harnstoffurethane folgende Monoaddukte gemäß der Patentschrift EP 1188779 hergestellt:
Monoaddukt 1 wird aus einem Gemisch von 2,4-Toluylendiisocyanat und 2,6-Toluylendiisocyanat (Desmodur T65, Bayer) und (Z)-Octadec-9-enol (Oleylalkohol, Merck) hergestellt.
Monoaddukt 2 wird aus einem Gemisch von 2,4-Toluylendiisocyanat und 2,6-Toluylendiisocyanat (Desmodur T65, Bayer) und Exxal™ 13 Tridecyl Alcohol (C₁₃-reiches, C₁₁₋₁₄-Alkanol; Exxon Mobil Corporation) hergestellt.
Monoaddukt 3 wird aus 2,4-Toluylendiisocyanat (Desmodur T100, Bayer) und 1-Dodecanol (Aldrich) hergestellt.

### Beispiel 1:

In einem Reaktionsgefäß mit Rührer, Tropftrichter und Stickstoff-Einlass werden unter Rühren 3,0 g (0,07 mol) LiCl in 150,0 g N-Methylpyrrolidon (BASF) gelöst. Danach werden 6,5 g (0,047 mol) meta-Xylylendiamin zugegeben und die Mischung auf 60°C erwärmt. Anschließend wird eine Mischung aus 24,3 g Monoaddukt 1 (0,052 mol) und 16,2 g Monoaddukt 2 (0,042 mol) unter Rühren innerhalb von 1 Stunde so zugetropft, so dass die Temperatur nicht über 80 °C steigt. Zur Vervollständigung der Reaktion wird das Reaktionsgemisch nach beendeter Zugabe für 3 Stunden bei 80 °C gerührt. Es wird ein klares, farbloses und flüssiges Produkt erhalten. Das Produkt enthält 25% Gehalt an Harnstoffurethan.

### Beispiel 2:

In einem Reaktionsgefäß mit Rührer, Tropftrichter und Stickstoff-Einlass werden unter Rühren 3,3 g (0,078 mol) LiCl in 90,0 g N-Octylpyrrolidon (BASF) gelöst.

Danach werden 7,2 g (0,052 mol) meta-Xylylendiamin zugegeben und die Mischung auf 60°C erwärmt. Anschließend werden 49,5 g (0,104 mol) Monoaddukt 1 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 40% Gehalt an Harnstoffurethan.

### Beispiel 3:

In einem Reaktionsgefäß mit Rührer, Tropftrichter und Stickstoff-Einlass werden unter Rühren 2,8 g (0,066 mol) LiCl in 150,0 g N-Octylpyrrolidon (BASF) gelöst. Danach werden 6,0 g (0,044 mol) meta-Xylylendiamin zugegeben und die Mischung auf 60°C erwärmt. Anschließend werden 41,2 g (0,088 mol) Monoaddukt 1 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 25% Gehalt an Harnstoffurethan.

### Beispiel 4:

In einem Reaktionsgefäß mit Rührer, Tropftrichter und Stickstoff-Einlass werden unter Rühren 2,8 g (0,066 mol) LiCl in 150,0 g N-Methylpyrrolidon (BASF) gelöst. Danach werden 6,0 g (0,044 mol) meta-Xylylendiamin zugegeben und die Mischung auf 60°C erwärmt. Anschließend werden 41,2 g (0,088 mol) Monoaddukt 1 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 25% Gehalt an Harnstoffurethan.

### Beispiel 5:

In einem Reaktionsgefäß mit Rührer, Tropftrichter und Stickstoff-Einlass werden unter Rühren 4,5 g (0,105 mol) LiCl in 225,0 g N-Octylpyrrolidon (BASF) gelöst. Danach werden 9,7 g (0,07 mol) meta-Xylylendiamin zugegeben und die Mischung auf 60°C erwärmt. Anschließend wird eine Mischung aus 36,5 g (0,077 mol) Monoaddukt 1 und 24,4 g (0,063 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 25% Gehalt an Harnstoffurethan.

### Beispiel 6:

In einem Reaktionsgefäß mit Rührer, Tropftrichter und Stickstoff-Einlass werden unter Rühren 5,4 g (0,127 mol) LiCl in 210,0 g Dimethylsulfoxid (DMSO, Merck) gelöst. Danach werden 11,7 g (0,085 mol) meta-Xylylendiamin zugegeben und die Mischung auf 60°C erwärmt. Anschließend wird eine Mischung aus 42,0 g (0,089 mol) Monoaddukt 1 und 31,0 g (0,081 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 30% Gehalt an Harnstoffurethan.

### Beispiel 7:

In einem Reaktionsgefäß mit Rührer, Tropftrichter und Stickstoff-Einlass werden unter Rühren 5,3 g (0,126 mol) LiCl in 210,0 g Dimethylsulfoxid (DMSO, Merck) gelöst. Danach werden 11,6 g (0,084 mol) meta-Xylylendiamin zugegeben und die Mischung auf 60°C erwärmt. Anschließend wird eine Mischung aus 45,6 g (0,097 mol) Monoaddukt 1 und 27,5 g (0,071 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 30% Gehalt an Harnstoffurethan.

### Beispiel 8:

In einem Reaktionsgefäß mit Rührer, Tropftrichter und Stickstoff-Einlass werden unter Rühren 6,3 g (0,149 mol) LiCl in 195,0 g Dimethylsulfoxid (DMSO, Merck) gelöst. Danach werden 13,7 g (0,099 mol) meta-Xylylendiamin zugegeben und die Mischung auf 60°C erwärmt. Anschließend wird eine Mischung aus 46,8 g (0,099 mol) Monoaddukt 1 und 38,2 g (0,099 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 35% Gehalt an Harnstoffurethan.

### Beispiel 9:

In einem Reaktionsgefäß mit Rührer, Tropftrichter und Stickstoff-Einlass werden unter Rühren 6,3 g (0,149 mol) LiCl in 195,0 g Dimethylsulfoxid (DMSO, Merck) gelöst. Danach werden 13,5 g (0,098 mol) meta-Xylylendiamin zugegeben und die Mischung auf 60°C erwärmt. Anschließend wird eine Mischung aus 51,1 g (0,108 mol) Monoaddukt 1 und 34,1 g (0,089 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 35% Gehalt an Harnstoffurethan.

### Beispiel 10:

In einem Reaktionsgefäß mit Rührer, Tropftrichter und Stickstoff-Einlass werden unter Rühren 6,2 g (0,146 mol) LiCl in 195,0 g Dimethylsulfoxid (DMSO, Merck) gelöst. Danach werden 13,4 g (0,098 mol) meta-Xylylendiamin zugegeben und die Mischung auf 60°C erwärmt. Anschließend wird eine Mischung aus 55,3 g (0,117 mol) Monoaddukt 1 und 30,1 g (0,078 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 35% Gehalt an Harnstoffurethan.

### Beispiel 11:

In einem Reaktionsgefäß mit Rührer, Tropftrichter und Stickstoff-Einlass werden unter Rühren 6,3 g (0,149 mol) LiCl in 245,0 g Dimethylsulfoxid (DMSO, Merck) gelöst. Danach werden 13,7 g (0,099 mol) meta-Xylylendiamin zugegeben und die Mischung auf 60°C erwärmt. Anschließend wird eine Mischung aus 46,8 g (0,099 mol) Monoaddukt 1 und 38,2 g (0,099 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 30% Gehalt an Harnstoffurethan.

### Beispiel 12:

In einem Reaktionsgefäß mit Rührer, Tropftrichter und Stickstoff-Einlass werden unter Rühren 6,3 g (0,149 mol) LiCl in 280,0 g Dimethylsulfoxid (DMSO, Merck) gelöst. Danach werden 13,7 g (0,099 mol) meta-Xylylendiamin zugegeben und die Mischung auf 60°C erwärmt. Anschließend wird eine Mischung aus 46,8 g (0,099 mol) Monoaddukt 1 und 38,2 g (0,099 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 27,5% Gehalt an Harnstoffurethan.

### Beispiel 13:

In einem Reaktionsgefäß mit Rührer, Tropftrichter und Stickstoff-Einlass werden unter Rühren 6,3 g (0,149 mol) LiCl in 280,0 g Dimethylsulfoxid (DMSO, Merck) gelöst. Danach werden 13,5 g (0,098 mol) meta-Xylylendiamin zugegeben und die Mischung auf 60°C erwärmt. Anschließend wird eine Mischung aus 51,1 g (0,108 mol) Monoaddukt 1 und 34,1 g (0,089 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 27,5% Gehalt an Harnstoffurethan.

### Beispiel 14:

In einem Reaktionsgefäß mit Rührer, Tropftrichter und Stickstoff-Einlass werden unter Rühren 6,2 g (0,146 mol) LiCl in 280,0 g Dimethylsulfoxid (DMSO, Merck) gelöst. Danach werden 13,4 g (0,098 mol) meta-Xylylendiamin zugegeben und die Mischung auf 60°C erwärmt. Anschließend wird eine Mischung aus 55,3 g (0,117 mol) Monoaddukt 1 und 30,1 g (0,078 mol) Monoaddukt 2 unter Rühren innerhalb von 1 Stunde zugetropft, so dass die Temperatur nicht über 80°C steigt. Zur Vervollständigung der Reaktion wird das Reaktionsgemisch für 3 Stunden bei 80°C gerührt. Es wird ein klares, farbloses und flüssiges Produkt erhalten. Das Produkt enthält 27,5% Gehalt an Harnstoffurethan.

### Beispiel 15:

In einem Reaktionsgefäß mit Rührer, Tropftrichter und Stickstoff-Einlass werden bei 60°C unter Rühren 4,2 g (0,099 mol) LiCl in 130,0 g in N-Octylpyrrolidon (BASF) gelöst. Danach werden 9,1 g (0,066 mol) meta-Xylylendiamin zugegeben, wobei die Lösung nach wenigen Sekunden trüb wird. Anschließend wird eine Mischung aus 32,1 g Monoaddukt 1 (0,066 mol) und 25,5 g Monoaddukt 3 (0,066 mol) unter Rühren innerhalb von 30 Minuten so zugetropft, dass die Temperatur nicht über 80 °C steigt. Während dieser Zeit klart das Reaktionsgemisch vollständig auf. Zur Vervollständigung der Reaktion wird das Reaktionsgemisch nach beendeter Zugabe für 3 Stunden bei 80 °C gerührt. Es wird ein klares, gelbliches und flüssiges Produkt erhalten. Das Produkt enthält 35% Harnstoffurethan.

### Beispiel 16:

In einem Reaktionsgefäß mit Rührer, Tropftrichter und Stickstoff-Einlass werden bei 60°C unter Rühren 4,0 g (0,095 mol) LiCl in 130,0 g in N-Octylpyrrolidon (BASF) gelöst. Danach werden 8,7 g (0,064 mol) meta-Xylylendiamin zugegeben, wobei die Lösung nach wenigen Sekunden trüb wird. Anschließend wird eine Mischung aus 45,0 g Monoaddukt 1 (0,095 mol) und 12,2 g Monoaddukt 3 (0,032 mol) unter Rühren innerhalb von 30 Minuten so zugetropft, dass die Temperatur nicht über 80 °C steigt. Während dieser Zeit klart das Reaktionsgemisch vollständig auf. Zur Vervollständigung der Reaktion wird das Reaktionsgemisch nach beendeter Zugabe für 3 Stunden bei 80 °C gerührt. Es wird ein klares, gelbliches und flüssiges Produkt erhalten. Das Produkt enthält 35% Harnstoffurethan.

### Beispiel 17 (Oligomer/Polymer; Mₙ = 3135, M_{w}=3708)

In einem Reaktionsgefäß mit Rührer, Tropftrichter und Stickstoff-Einlass werden bei 60°C unter Rühren 6,0 g (0,142 mol) LiCl in 130,0 g in N-Octylpyrrolidon (BASF) gelöst. Danach werden 9,7 g (0,071 mol) meta-Xylylendiamin zugegeben, wobei die Lösung nach wenigen Sekunden trüb wird. Anschließend wird eine Mischung aus 51,4 g Monoaddukt 1 (0,109 mol) und 2,8 g Toluylendiisocyanat (0,016 mol) (Desmodur T80; Gemisch aus 2,4-Toluylendiisocyanat und 2,6-Toluylendiisocyanat von Bayer) unter Rühren innerhalb von 30 Minuten so zugetropft, dass die Temperatur nicht über 80 °C steigt. Während dieser Zeit klart das Reaktionsgemisch vollständig auf. Zur Vervollständigung der Reaktion wird das Reaktionsgemisch nach beendeter Zugabe für 3 Stunden bei 80 °C gerührt. Es wird ein klares, gelbliches und flüssiges Produkt erhalten. Das Produkt enthält 35% Harnstoffurethan.

### Beispiel 18 (Oligomer/Polymer; Mₙ=3317, M_{w}=3967)

In einem Reaktionsgefäß mit Rührer, Tropftrichter und Stickstoff-Einlass werden unter Rühren 6,2 g (0,147 mol) LiCl in 130,0 g N-Octylpyrrolidon (Aldrich) gelöst. Danach werden 10,0 g (0,073 mol) meta-Xylylendiamin zugegeben und die Mischung auf 60°C erwärmt. Anschließend wird eine Mischung aus 50,1 g (0,105 mol) Monoaddukt 1 und 3,6 g (0,021 mol) Toluylendiisocyanat (Isomerengemisch 2,4/2,6-Toluylendiisocyanat im Verhältnis 4:1 von Bayer; Desmodur T80) unter Rühren innerhalb von 35 Minuten zugetropft, so dass die Temperatur nicht über 80°C steigt. Zur Vervollständigung der Reaktion wird das Reaktionsgemisch für 3 Stunden bei 80°C und noch für 30 Minuten bei 90°C gerührt. Es wird ein klares, gelbes und flüssiges Produkt erhalten. Das Produkt enthält 35% Gehalt an Harnstoffurethan.

### Beispiel 19 (Oligomer/Polymer; Mₙ=3377, M_{w}=4102)

In einem Reaktionsgefäß mit Rührer, Tropftrichter und Stickstoff-Einlass werden unter Rühren 6,5 g (0,153 mol) LiCl in 130,0 g N-Octylpyrrolidon (Aldrich) gelöst. Danach werden 10,4 g (0,077 mol) meta-Xylylendiamin zugegeben und die Mischung auf 60°C erwärmt. Anschließend wird eine Mischung aus 48,7 g (0,102 mol) Monoaddukt 1 und 4,4 g (0,026 mol) Toluylendiisocyanat (Isomerengemisch 2,4/2,6-Toluylendiisocyanat im Verhältnis 4:1 von Bayer; Desmodur T80) unter Rühren innerhalb von 35 Minuten zugetropft, so dass die Temperatur nicht über 80°C steigt. Zur Vervollständigung der Reaktion wird das Reaktionsgemisch für 3 Stunden bei 80°C und noch für 30 Minuten bei 90°C gerührt. Es wird ein klares, gelbes und flüssiges Produkt erhalten. Das Produkt enthält 35% Gehalt an Harnstoffurethan.

### Beispiel 20 (Oligomer/Polymer; Mₙ=5021, M_{w}=7865)

In einem Reaktionsgefäß mit Rührer, Tropftrichter und Stickstoff-Einlass werden bei 60°C unter Rühren 4,8 g (0,114 mol) LiCl in 130,0 g N-Octylpyrrolidon (Aldrich) gelöst. Danach werden 15,5 g (0,114 mol) meta-Xylylendiamin zugegeben, wobei die Lösung nach wenigen Sekunden trüb wird. Anschließend wird eine Mischung aus 36,4 g Monoaddukt 1 (0,076 mol) und 13,3 g Toluylendiisocyanat (0,076 mol) (Isomerengemisch 2,4/2,6-Toluylendiisocyanat im Verhältnis 4:1 von Bayer; Desmodur T80) unter Rühren innerhalb von 50 Minuten so zugetropft, dass die Temperatur nicht über 80 °C steigt. Während dieser Zeit klart das Reaktionsgemisch fast komplett auf. Zur Vervollständigung der Reaktion wird das Reaktionsgemisch nach beendeter Zugabe für 3 Stunden bei 80 °C gerührt. In dieser Zeit klart die Lösung vollständig auf, sodass ein klares, gelbes und viskoses Produkt erhalten wird. Das Produkt enthält 35% Harnstoffurethan.

### Beispiel 21 (Oligomer/Polymer; Mₙ=3935, M_{w}=5512)

In einem Reaktionsgefäß mit Rührer, Tropftrichter und Stickstoff-Einlass werden bei 60°C unter Rühren 4,0 g (0,094 mol) LiCl in 130,0 g N-Octylpyrrolidon (Aldrich) gelöst. Danach werden 12,8 g (0,094 mol) meta-Xylylendiamin zugegeben, wobei die Lösung nach wenigen Sekunden trüb wird. Anschließend wird eine Mischung aus 45,0 g Monoaddukt 1 (0,094 mol) und 8,2 g Toluylendiisocyanat (0,047 mol) (Isomerengemisch 2,4/2,6-Toluylendiisocyanat im Verhältnis 4:1 von Bayer; Desmodur T80) unter Rühren innerhalb von 50 Minuten so zugetropft, dass die Temperatur nicht über 80 °C steigt. Während dieser Zeit klart das Reaktionsgemisch komplett auf. Zur Vervollständigung der Reaktion wird das Reaktionsgemisch nach beendeter Zugabe für 3 Stunden bei 80 °C gerührt. Es wird ein klares, gelbes und viskoses Produkt erhalten. Das Produkt enthält 35% Harnstoffurethan.

### (II) Anwendungstechnische Prüfung der als Rheologiesteuerungsmittel geeigneten Harnstoffurethane

**Eingesetzte Rohstoffe**

| **Name** | **Beschreibung** | **Hersteller** |
|---|---|---|
| Acridic A 188 | Nicht-wässrige Dispersion | Dainippon Ink & Chemicals, Inc. |
| Acridic A 1300 | Nicht-wässrige Dispersion | Dainippon Ink & Chemicals, Inc. |
| Bayferrox 130 M | Eisenoxidrotpigment | Lanxess Deutschland GmbH |
| Blanc Fixe N | Bariumsulfat | Sachtleben Chemie GmbH |
| Borchinox M 2 | 2-Butanonoxim | Borchers GmbH |
| BYK-052 | Entschäumer | BYK-Chemie GmbH |
| Claytone 3 | Schichtsilikat | BYK Chemie GmbH |
| Disperbyk-108 | Netz- und Dispergieradditiv | BYK-Chemie GmbH |
| Dowanol PMA | 1,2-Propanediol monoacetat - Monomethylether | Dow Chemical Company |
| Durcal 5 | Calciumcarbonat | Omya |
| Heucophos ZCP - plus | Zink-Calcium-Strontium-Aluminium-Orthophosphat-Silikathydrat | Heubach GmbH |
| Heucorin RZ | Org.Korrosionsinhibitor Zink-5-nitro-Isophthalat | Heubach GmbH |
| Isomerized C1618 Alpha Olefin | Synthetisches Olefin | Ineos Oligomers |
| Micro Talc AT-1 | Talk-Magnesite | Mondo Minerals BV |
| Octa Soligen Calcium 10 | Calcium Sikkativ | Borchers GmbH |
| Octa Soligen Cobalt 12 | Kobalt Sikkativ | Borchers GmbH |
| Setal A F 26 X | Kurzölalkyd 60%ig in Xylol | Nuplex Resins GmbH |
| Shellsol A | Aromatisches Kohlenwasserstoffgemisch | Overlack AG |
| Testbenzin K 30 | Testbenzin 145-200 | Overlack AG |
| Tioxide TR 92 | Titandioxid | Huntsman Pigments |
| Worléekyd B 6301 | Langöliges Alkydharz, 90%ig in dearomat. HC 180-220 | Worlée Chemie GmbH |
| Xylol | Isomerengemisch | Overlack AG |

**Erläuterung der Bewertungsskala**

| | | |
|---|---|---|
| Gelstärke: | 1 | sehr stark |
| | 2 | stark |
| | 3 | mittel |
| | 4 | sehr schwach |
| | 5 | kein Gel |
| Trübung (Verträglichkeit) : | 1 | klar |
| | 2 | leicht trüb |
| | 3 | trüb |
| | 4 | stark trüb |
| | 5 | sehr stark trüb |

### Prüfsystem 1: Lösungsmittelmischungen

In einer 100 ml-Glasflasche werden 50 g der jeweiligen Lösungsmittelmischung bestehend aus Xylol/n-Butanol 90:10 (w/w) und Dowanol PMA/Shellsol A/Iso-Butanol 50:25:25 (w/w/w) vorgelegt und anschließend das jeweilige Additiv unter Rühren mit dem Dispermat CV (Zahnscheibe d = 2,5 cm bei 1000 U/min) eingearbeitet. Es wurde dabei in allen Fällen eine Dosierung gewählt, welche 2% des Harnstoffurethans (bezogen auf die gesamte Masse an Lösungsmittelmischung) entspricht. Nach erfolgter Zugabe wird noch 1 Minute weitergerührt.

Anschließend lässt man die Proben 1 Tag bei Raumtemperatur stehen und beurteilt anschließend visuell die Gelstärke als Maß für die rheologische Wirksamkeit und die Verträglichkeit des Additivs im System anhand der Trübung.

| **Produkt** | **Xylol / n-Butanol 90:10** | | **Dowanol MPA/Shellsol A/ Iso-Butanol 50:25:25** | |
|---|---|---|---|---|
| | Gelstärke | Trübung | Gelstärke | Trübung |
| Nullprobe ohne Additiv | 5 | 1 | 5 | 1 |
| Vergleichsbeispiel 1 | 4 | 3 | 5 | 4 |
| Beispiel 1 | 2 | 2 | 3 | 3 |
| Beispiel 2 | 3 | 1 | 3 | 1 |

Aus der Tabelle ist erkennbar, dass das Vergleichsbeispiel 1 eine deutlich schlechtere Gelstärke aufbaut und unverträglicher ist als die erfindungsgemäßen Beispiele 1 und 2.

### Prüfsystem 2: Acrydic A-188 / A-1300-Weißlack

In einer 100 ml-Glasflasche werden 50 g Acrydic A-188 / A-1300-Weißlack gemäß der u.a. Zusammensetzung vorgelegt und anschließend das jeweilige Additiv unter Rühren mit dem Dispermat CV (Zahnscheibe d = 2,5 cm bei 1000 U/min) eingearbeitet. Nach erfolgter Zugabe wird noch 1 Minute weitergerührt. Es wurde dabei in allen Fällen eine Dosierung gewählt, welche 0,25 Gew.% des Harnstoffurethans (bezogen auf die gesamte Masse der Formulierung) entspricht. Anschließend lässt man die Proben 1 Tag bei Raumtemperatur stehen und führt anschließend die Prüfung des Standvermögens als Maß für die rheologische Wirksamkeit unter Applikationsbedingungen durch.

Hierzu wird die Probe mit dem Spatel gleichmäßig durchgerührt und dann mit einer Stufenrakel 50-500 µm und einer automatischen Aufziehbank (Fa. BYK-Gardner) bei einer Geschwindigkeit von 5 cm/s auf Kontrastkarten appliziert. Nach der Applikation werden die Kontrastkarten direkt horizontal zur Trocknung aufgehängt. Nach der Trocknung wird die maximale Schichtdicke in µm (naß) bestimmt, bei der der Lack nicht abläuft, das heißt keine Läufer oder Wulstbildung erkennbar ist. Je höher der Wert für das Standvermögen bei Einsatz gleicher Mengen an Harnstoffurethan ist, desto besser ist die rheologische Wirksamkeit.

Formulierung des Weißlacks:

**Acrydic A-188 / A-1300-Weißlack**

| | |
|---|---|
| Acridic A 188 | 12,7 |
| Tioxide TR 92 | 22,0 |
| Disperbyk 108 | 2,5 |
| Testbenzin K 30 | 12,5 |
| Dispermat, 40°C, 30 min, 8500 U/min, 4 cm-Zahnscheibe | |
| Acridic A 1300 | 44,0 |
| Testbenzin K 30 | 6,3 |
| | 100,0 |

**Ergebnisse:**

| **Produkt** | **Standvermögen µm (naß)** |
|---|---|
| Nullprobe ohne Additiv | 100 |
| Vergleichsbeispiel 2 | 150 |
| Beispiel 3 | 200 |
| Beispiel 4 | 200 |

In der Tabelle ist erkennbar, dass das Vergleichsbeispiel 2 eine schlechtere rheologische Wirksamkeit in Form von Standvermögen zeigt als die erfindungsgemäßen Beispiele 3 und 4.

### Prüfsystem 3: Setal A F 26 X Weißlack

In einer 100 ml-Glasflasche werden 50 g Setal A F 26 X Weißlack gemäß der u.a. Zusammensetzung vorgelegt und anschließend das jeweilige Additiv unter Rühren mit dem Dispermat CV (Zahnscheibe d = 2,5 cm bei 1000 U/min.) eingearbeitet. Es wurde dabei in allen Fällen eine Dosierung gewählt, welche 0,5 Gew.% des Harnstoffurethans (bezogen auf die gesamte Masse der Lackformulierung) entspricht. Nach erfolgter Zugabe wird noch 1 Minute weitergerührt.

Anschließend lässt man die Proben 1 Tag bei Raumtemperatur stehen und beurteilt durch Applikation das Standvermögen als Maß der rheologischen Wirksamkeit. Hierzu wird die Probe mit dem Spatel gleichmäßig durchgerührt und dann mit einer Stufenrakel 50-500 µm und einer automatischen Aufziehbank (Fa. BYK Gardner) bei einer Geschwindigkeit von 5 cm/s auf Kontrastkarten appliziert. Nach der Applikation werden die Kontrastkarten direkt horizontal zur Trocknung aufgehängt. Nach der Trocknung wird die max. Schichtdicke in µm (naß) bestimmt, bei der der Lack nicht abläuft, das heißt keine Läufer oder Wulstbildung erkennbar ist. Je höher der Wert für das Standvermögen bei Einsatz gleicher Mengen des Harnstoffurethans ist, desto besser ist die rheologische Wirksamkeit.

**Formulierung des Setal-Weißlackes:**

| | |
|---|---|
| Setal A F 26 X | 34,5 |
| Shellsol A | 7,8 |
| Emulgierhilfsmittel* | 0,2 |
| BYK-052 | 0,2 |
| Bayferrox 130 M | 6,0 |
| Micro Talc AT-1 | 7,0 |
| Heucophos ZCP - plus | 21,0 |
| Heucorin RZ | 0,5 |
| Dispermat, 40°C, 30 min, 8500 U/min, 4 cm-Zahnscheibe | |
| Setal A F 26 X | 8,0 |
| Shellsol A | 14,2 |
| Calcium 10 | 0,3 |
| Cobalt 12 | 0,2 |
| Borchinox M 2 | 0,1 |
| | 100,0 |

| | |
|---|---|
| *erhältlich von der BYK Chemie GmbH | |

**Ergebnisse:**

| **Produkt** | **Standvermögen µm (naß)** |
|---|---|
| Nullprobe ohne Additiv | 50 |
| Vergleichsbeispiel 2 | 100 |
| Beispiel 1 | 200 |
| Beispiel 3 | 200 |
| Beispiel 5 | 200 |

Aus der Tabelle ist erkennbar, dass das Vergleichsbeispiel 2 ein schlechteres Standvermögen ermöglicht als die erfindungsgemäßen Produkte.

### Prüfsystem 4: Worleekyd B6301 Bindemittel

In einer 100 ml-Glasflasche werden 50 g Worleekyd B6301 Bindemittel vorgelegt und anschließend das jeweilige Additiv unter Rühren mit dem Dispermat CV (Zahnscheibe d = 2,5 cm bei 1000 U/min.) eingearbeitet. Es wurde dabei in allen Fällen eine Dosierung gewählt, welche 1,0 Gew.% des Harnstoffurethans (bezogen auf die gesamte Masse der Lackformulierung) entspricht. Nach erfolgter Zugabe wird noch 1 Minute weitergerührt.

Anschließend lässt man die Proben 1 Tag bei Raumtemperatur stehen und beurteilt durch Applikation das Standvermögen als Maß der rheologischen Wirksamkeit. Hierzu wird die Probe mit dem Spatel gleichmäßig durchgerührt und dann mit einer Stufenrakel 50-500 µm und einer automatischen Aufziehbank (Fa. BYK-Gardner) bei einer Geschwindigkeit von 5 cm/s auf Kontrastkarten appliziert. Nach der Applikation werden die Kontrastkarten direkt horizontal zur Trocknung aufgehängt. Nach der Trocknung wird die max. Schichtdicke in µm (naß) bestimmt, bei der der Lack nicht abläuft, das heißt keine Läufer oder Wulstbildung erkennbar ist. Je höher der Wert für das Standvermögen bei Einsatz gleicher Wirksubstanz ist, desto besser ist die rheologische Wirksamkeit.

| **Produkt** | **Standvermögen µm (naß)** |
|---|---|
| Nullprobe ohne Additiv | < 50 |
| Vergleichsbeispiel 1 | 50 |
| Vergleichsbeispiel 2 | 100 |
| Beispiel 6 | 500 |
| Beispiel 7 | 500 |
| Beispiel 8 | 600 |
| Beispiel 9 | 550 |
| Beispiel 10 | 550 |
| Beispiel 11 | 350 |
| Beispiel 12 | 350 |
| Beispiel 13 | 400 |
| Beispiel 14 | 350 |

Aus der Tabelle ist erkennbar, dass die Vergleichsbeispiele 1 und 2 ein schlechteres Standvermögen ermöglichen als die erfindungsgemäßen Beispiele.

### Prüfsystem 5: Ölbasierender Bohrschlamm

Zunächst werden 400 g Bohrschlamm mittels eines Hamiton Beach-Mixers, Typ GM20, Modell HMD200-CE (Hersteller: Hamilton Beach; Einstellung: Stufe 1) nach der angegebenen Rezeptur hergestellt.

Der Schlamm wird in 100 g-Proben aufgeteilt und das jeweilige Additiv 5 min mit einem Ultra-Turrax-Rührer (Hersteller: IKA-Werke GmbH, Modell T 45) bei 6000 U/min eingearbeitet (die Nullprobe wird analog geschert). Es wurde dabei in allen Fällen eine Dosierung gewählt, welche 0,5 Gew.% des Harnstoffurethans (bezogen auf die gesamte Masse des Bohrschlamms) entspricht.

Für das Separationsverhalten wurden jeweils 60 g Bohrschlamm in 50 ml-Schnappdeckelgläser abgefüllt und bei Raumtemperatur 4 Wochen gelagert.

Danach erfolgt die Auswertung der Separation (Synerese-Bildung) in % bezogen auf die Gesamtfüllhöhe im Schnappdeckelglas als Maß für die rheologische Wirksamkeit. Je geringer die Höhe der separierten Phase, desto besser ist die rheologische Wirksamkeit des Additivs und desto besser lässt sich dieses folglich gegen die Separation der Komponenten einsetzen.

Für ausgewählte Beispiele werden die restlichen 40 g des Bohrschlamms in 50 ml-Glasflaschen abgefüllt. Nach 16 h Standzeit bei RT erfolgt die Messung der Viskosität temperaturabhängig auf dem Rheometer Physica MCR-301 (Hersteller: Anton Paar GmbH) bei den drei Temperaturen 4°C, 25°C und 66°C scherratengesteuert bei den Scherraten 10, 150, 300, 500 und 1000 1/s mit einer Platte-Platte Geometrie DPP30 und 0,5 mm Messspalt.

**Ölbasierender Bohrschlamm:**

| Komponente | Einwaage/g | Mischung/min |
|---|---|---|
| Isomerized C1618 Alpha Olefin | 25,0 | |
| Claytone 3 | 0,2 | 10 |
| Durcal 5 | 0,5 | 10 |
| Emulgierhilfsmittel* | 1,8 | 10 |
| CaCl₂-Lösung 25%ig in Wasser | 18,5 | 10 |
| Blanc Fixe N | 54,0 | 10 |
| | 100,0 | |

| | | |
|---|---|---|
| *erhältlich von der BYK Chemie GmbH | | |

**Ergebnisse: Separation**

| **Produkt** | **Dosierung Gew.% Harnstoffurethan** | **Separation (Synerese) % nach 4 Wochen RT** |
|---|---|---|
| Nullprobe ohne Additiv | - | 36 |
| Vergleichsbeispiel 1 | 0,5 | 27 |
| Vergleichsbeispiel 3 | 0,5 | 44 |
| Beispiel 2 | 0,5 | 9 |
| Beispiel 15 | 0,5 | 4 |
| Beispiel 16 | 0,5 | 3 |
| Beispiel 17 | 0,5 | 1 |
| Beispiel 18 | 0,5 | 1 |
| Beispiel 19 | 0,5 | 1 |
| Beispiel 20 | 0,5 | 4 |
| Beispiel 21 | 0,5 | 2 |

An der Tabelle ist erkennbar, dass die erfindungsgemäßen Produkte gemäß der Beispiele 2 und 15 bis 21 das System besser gegen Separation zu stabilisieren vermögen als die Vergleichsbeispiele 1 und 3 oder die Nullprobe.

**Ergebnisse: Viskositätsmessung**

| **Produkt** | **Temperatur/°C** | **Viskosität/ mPas bei Scherrate 1/s** | | | | |
|---|---|---|---|---|---|---|
| | | **10** | **150** | **300** | **500** | **1000** |
| Nullprobe ohne Additiv | 4,4 | 797 | 178 | 133 | 114 | 92 |
| | 25 | 272 | 79 | 62 | 55 | 46 |
| | 66 | 62 | 19 | 16 | 14 | 12 |
| Vergleichsbeispiel 1 | 4,4 | 831 | 218 | 171 | 152 | 130 |
| | 25 | 305 | 101 | 83 | 76 | 67 |
| | 66 | 183 | 49 | 39 | 34 | 28 |
| Beispiel 2 | 4,4 | 2953 | 460 | 308 | 247 | 182 |
| | 25 | 1987 | 289 | 189 | 149 | 108 |
| | 66 | 1176 | 177 | 117 | 100 | 73 |

Aus der Tabelle ist erkennbar, dass das Vergleichsbeispiel 1 nur eine geringere Viskositätserhöhung ermöglicht, im Vergleich zum erfindungsgemäßen Produkt des Beispiels 2.

## Patentansprüche

1. Harnstoffurethane der allgemeinen Formel (I) 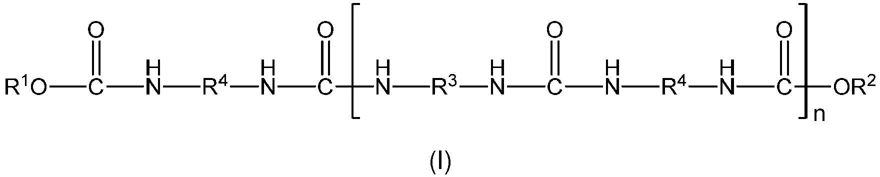 worin
R¹ für einen einfach oder mehrfach ungesättigten, verzweigten oder unverzweigten Alkenylrest mit 12 bis 24 Kohlenstoffatomen, einen einfach oder mehrfach ungesättigten, verzweigten oder unverzweigten Alkinylrest mit 12 bis 24 Kohlenstoffatomen oder für einen mehrfach ungesättigten Kohlenwasserstoff-Rest mit 12 bis 24 Kohlenstoffatomen, der mindestens eine Kohlenstoff-Kohlenstoff-Doppelbindung und mindestens eine Kohlenstoff-Kohlenstoff-Dreifachbindungen aufweist, steht,
R² für einen gesättigten, verzweigten oder unverzweigten Alkylrest mit 8 bis 24 Kohlenstoffatomen oder für einen einfach oder mehrfach ungesättigten, verzweigten oder unverzweigten Alkenylrest mit 12 bis 24 Kohlenstoffatomen, einen einfach oder mehrfach ungesättigten, verzweigten oder unverzweigten Alkinylrest mit 12 bis 24 Kohlenstoffatomen oder für einen mehrfach ungesättigten Kohlenwasserstoff-Rest mit 12 bis 24 Kohlenstoffatomen, der mindestens eine Kohlenstoff-Kohlenstoff-Doppelbindung und mindestens eine Kohlenstoff-Kohlenstoff-Dreifachbindungen aufweist, steht, und
alle n Reste R³ unabhängig voneinander für einen oder mehrere Reste gewählt aus den Struktureinheiten (IIa-o), (IIa-m), (IIa-p), (IIb-o), (IIb-m) und (IIb-p) 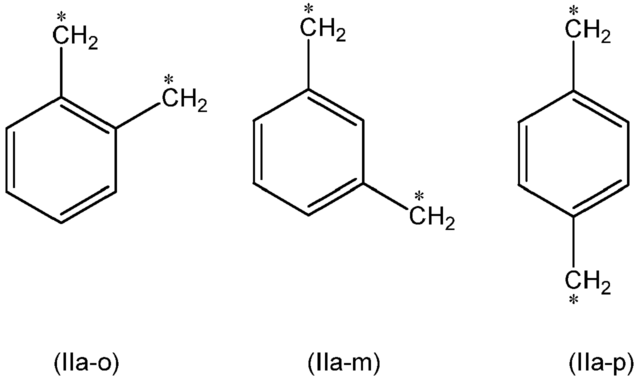 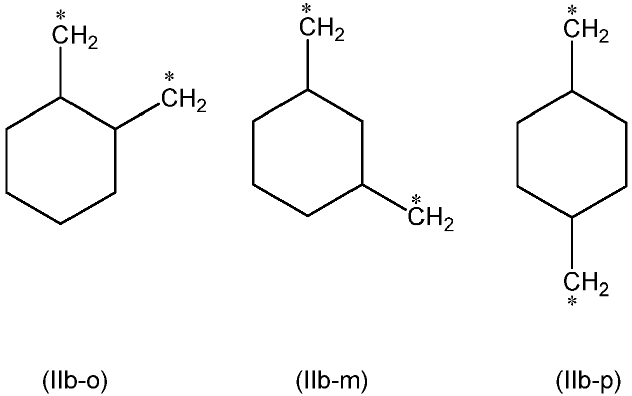
stehen, und
alle n+1 Reste R⁴ unabhängig voneinander für einen oder mehrere Reste gewählt aus den Struktureinheiten (IIIa), (IIIb), (IIIc), (IIId), (IIIe), (IIIf), (IIIg) und (IIIh) 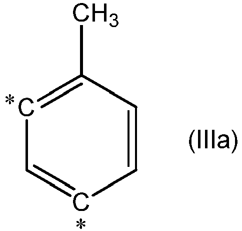 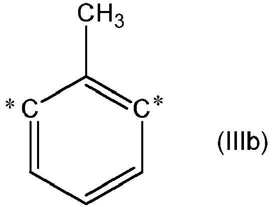 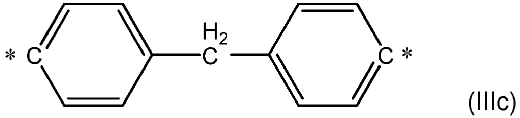 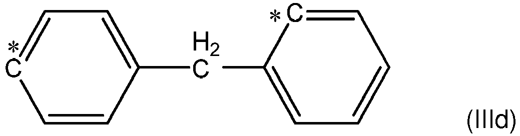 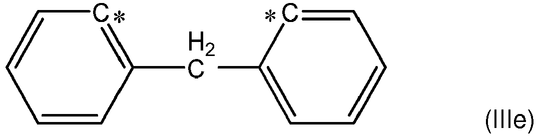 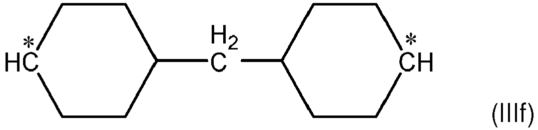 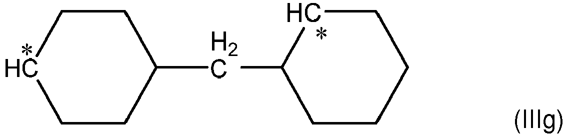 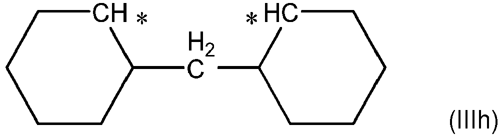
stehen, und
n für eine ganze Zahl ≥ 1 steht, wobei sich die Obergrenze für n aus dem maximalen zahlenmittleren Molekulargewicht Mₙ der Harnstoffurethane der allgemeinen Formel (I) ergibt, welches 65000 g/mol beträgt und welches mittels Gelpermeationschromatographie nach DIN 55672-2 unter Verwendung eines Polymethylmethacrylat-Standards bestimmt wird, und
wobei die Bindungsstellen der Reste R³ und R⁴ an die benachbarten NH-Gruppen in obigen Formeln durch das Sternsymbol * gekennzeichnet sind.

2. Harnstoffurethane gemäß Anspruch 1, **dadurch gekennzeichnet, dass**
der Rest R¹ für einen einfach ungesättigten Alkenylrest mit 16 bis 20 Kohlenstoffatomen steht,
der Rest R² für einen gesättigten, verzweigten Alkylrest mit 10 bis 16 Kohlenstoffatomen oder für einen einfach ungesättigten Alkenylrest mit 16 bis 20 Kohlenstoffatomen steht,
die n Reste R³ unabhängig voneinander für einen oder mehrere Reste gewählt aus den Struktureinheiten (IIa-m) und (IIa-p) stehen, und
die n+1 Reste R⁴ unabhängig voneinander für einen oder mehrere Reste der Struktureinheiten (IIIa) und (IIIb) stehen.

3. Harnstoffurethane gemäß einem der Ansprüche 1 oder 2, **dadurch gekennzeichnet, dass**
der Rest R¹ für einen unverzweigten Octadecenylrest steht,
der Rest R² für einen verzweigten oder unverzweigten C₁₀-C₁₄-Alkylrest oder für einen unverzweigten Octadecenylrest steht,
die n Reste R³ für einen Rest der Struktureinheit (IIa-m) stehen, und
die n+1 Reste R⁴ unabhängig voneinander für einen oder mehrere Reste der Struktureinheiten (IIIa) und (IIIb) stehen.

4. Harnstoffurethane gemäß einem oder mehreren der Ansprüche 1 bis 3, **dadurch gekennzeichnet, dass**
in den n+1 Resten R⁴ die Struktureinheiten (IIIa) und (IIIb) vorzugsweise in einem molaren Verhältnis von 40 zu 60 bis zu einem molaren Verhältnis von 100 zu 0 stehen.

5. Harnstoffurethane gemäß einem oder mehreren der Ansprüche 1 bis 4, **dadurch gekennzeichnet, dass**
R¹ und R² für einen einfach oder mehrfach ungesättigten, verzweigten oder unverzweigten Alkenylrest mit 16 bis 20 Kohlenstoffatomen stehen.

6. Harnstoffurethane gemäß einem oder mehreren der Ansprüche 1 bis 5, **dadurch gekennzeichnet, dass** R¹ für Oleyl steht.

7. Harnstoffurethane gemäß einem oder mehreren der Ansprüche 1 bis 6, **dadurch gekennzeichnet, dass** R¹ = R² ist.

8. Harnstoffurethan-Zusammensetzungen, enthaltend eines oder mehrere der Harnstoffurethane der Ansprüche 1 bis 7, **dadurch gekennzeichnet, dass**
der Gewichts-Anteil der Harnstoffurethane der Formel (I), bei welchen beide Reste R¹ und R² ungesättigt sind, 10% bis 100%, bezogen auf die Gesamtheit der Harnstoffurethane der Formel (I), beträgt, und
der Gewichts-Anteil der Harnstoffurethane der Formel (I), bei welchen nur der Rest R¹ ungesättigt ist, 0% bis 90%, bezogen auf die Gesamtheit der Harnstoffurethane der Formel (I), beträgt.

9. Harnstoffurethan-Zusammensetzung gemäß Anspruch 8, **dadurch gekennzeichnet, dass** diese flüssig ist.

10. Harnstoffurethan-Zusammensetzung gemäß einem der Ansprüche 8 oder 9, wobei das oder die Harnstoffurethane in einem polaren, aprotischen Lösemittel gelöst vorliegen.

11. Harnstoffurethan-Zusammensetzung gemäß einem oder mehreren der Ansprüche 8 bis 10, wobei das polare, aprotische Lösemittel gewählt ist aus der Gruppe bestehend aus substituierten oder unsubstituierten N-Alkylpyrrolidonen, Dialkylsulfoxiden, substituierten oder unsubstituierten Amiden und organischen Salzen mit einem Schmelzpunkt ≤ 80°C.

12. Verfahren zur Herstellung der Harnstoffurethan-Zusammensetzungen gemäß einem oder mehreren der Ansprüche 8 bis 11, **dadurch gekennzeichnet, dass** in Gegenwart eines polaren aprotischen Lösemittels
(a) ein oder mehrere Monoaddukte der Formel R'-O-(CO)-NH-R⁴-NCO oder der Formel R²-O-(CO)-NH-R⁴-NCO aus einem oder mehreren Monoalkoholen der Formel R¹OH und/oder R²OH und einem oder mehreren Diisocyanaten der Formel OCN-R⁴-NCO hergestellt werden und dieses Monoaddukt oder diese Monoaddukte anschließend
(i) mit einem oder mehreren Diaminen der Formel H₂N-R³-NH₂ oder
(ii) mit einem oder mehreren beidseitig NH₂-terminerten Präpolymeren aus einem oder mehreren Diaminen der Formel H₂N-R³-NH₂ und einem oder mehreren Diisocyanaten der Formel OCN-R⁴-NCO oder
(iii) mit einem oder mehreren Diaminen der Formel H₂N-R³-NH₂ sowie einem oder mehreren Diisocyanaten der Formel OCN-R⁴-NCO oder
(iv) mit einem oder mehreren beidseitig NH₂-terminierten Präpolymeren oder einer Mischung aus einem oder mehreren Diaminen der Struktur H₂N-R³-NH₂ und einem oder mehreren beidseitig NH₂-terminierten Präpolymeren, sowie einem oder mehreren Diisocyanaten der Formel OCN-R⁴-NCO
umgesetzt wird; oder
(b) ein oder mehrere beidseitig NCO-terminierte Präpolymere aus einem oder mehreren Diisocyanaten der Formel OCN-R⁴-NCO und einem oder mehreren Diaminen der Formel H₂N-R³-NH₂ hergestellt wird beziehungsweise werden, und das oder die Präpolymere mit einem oder mehreren Monoalkoholen der Formeln R¹OH und/oder R²OH umgesetzt wird beziehungsweise werden;
und
wobei in den Verfahrensvarianten (a) und (b) mindestens ein Monoalkohol der Formel R¹OH eingesetzt wird.

13. Verfahren zur Herstellung eines Harnstoffurethans (I) wie es in den Ansprüchen 1 bis 7 definiert ist, **dadurch gekennzeichnet, dass** das Verfahren nach Anspruch 12 durchgeführt wird und anschließend das polare aprotische Lösemittel entfernt wird.

14. Verwendung der Harnstoffurethane (I) wie sie in den Ansprüchen 1 bis 7 definiert sind oder der Harnstoffurethan-Zusammensetzungen wie sie in den Ansprüchen 8 bis 11 definiert sind oder der Harnstoffurethan-Zusammensetzungen erhältlich nach einem der in den Ansprüchen 12 und 13 beschriebenen Verfahren als Rheologiesteuerungsmittel und/oder Antiabsetzmittel.

15. Rheologiesteuerungs- und/oder Antiabsetzmittel, **dadurch gekennzeichnet, dass** diese ein oder mehrere Harnstoffurethane (I) wie sie in den Ansprüchen 1 bis 7 definiert sind oder eine oder mehrere der Harnstoffurethan-Zusammensetzungen wie sie in den Ansprüchen 8 bis 11 definiert sind, enthält.

16. Flüssige Formulierung gewählt aus der Gruppe bestehend aus Beschichtungsmitteln, Kunststoffformulierungen, Pigmentpasten, Dichtstoffformulierungen, Kosmetika, Keramikformulierungen, Bohrspüllösungen, nicht-wässrigen Slurries, Klebstoffformulierungen, Vergussmassen, Baustoffformulierungen, Schmierstoffen, Spachtelmassen, Reinigungsmitteln, Druckfarben und Tinten, **dadurch gekennzeichnet, dass** diese ein oder mehrere Harnstoffurethane (I) wie sie in den Ansprüchen 1 bis 7 definiert sind oder eine oder mehrere der Harnstoffurethan-Zusammensetzungen wie sie in den Ansprüchen 8 bis 11 definiert sind, enthalten.

17. Flüssige Formulierung nach Anspruch 16, wobei der Anteil des Harnstoffurethans (I) beziehungsweise der Harnstoffurethane (I) in der gesamten flüssigen Formulierung 0,1 bis 5 Gew.% beträgt.

18. Flüssige Formulierung gemäß einem der Ansprüche 16 oder 17, **dadurch gekennzeichnet, dass** in der flüssigen Formulierung zusätzlich mindestens ein Kohlenwasserstoff ausgewählt aus der Gruppe der aliphatischen, cycloaliphatischen, aromatischen und aralphatischen Kohlenwasserstoffe enthalten ist.

19. Flüssige Formulierung gemäß Anspruch 18, wobei der Kohlenwasserstoff oder die Kohlenwasserstoffe zu mindestens 10 Gew.-% bezogen auf das Gesamtgewicht der flüssigen Formulierung in der flüssigen Formulierung enthalten ist beziehungsweise sind.

## Claims

1. Urea urethane of the general formula (I) 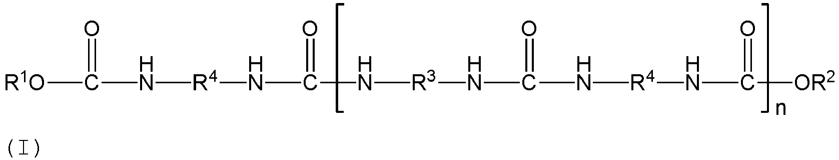 in which
R¹ is a mono- or polyunsaturated, branched or unbranched alkenyl radical having 12 to 24 carbon atoms, a mono- or polyunsaturated, branched or unbranched alkynyl radical having 12 to 24 carbon atoms or a polyunsaturated hydrocarbyl radical which has 12 to 24 carbon atoms and has at least one carbon-carbon double bond and at least one carbon-carbon triple bond,
R² is a saturated branched or unbranched alkyl radical having 8 to 24 carbon atoms or a mono- or polyunsaturated, branched or unbranched alkenyl radical having 12 to 24 carbon atoms, a mono- or polyunsaturated, branched or unbranched alkynyl radical having 12 to 24 carbon atoms or a polyunsaturated hydrocarbyl radical which has 12 to 24 carbon atoms and has at least one carbon-carbon double bond and at least one carbon-carbon triple bond, and all n R³ radicals are independently one or more radicals selected from the structural units (IIa-o), (IIa-m), (IIa-p), (IIb-o), (IIb-m) and (IIb-p) 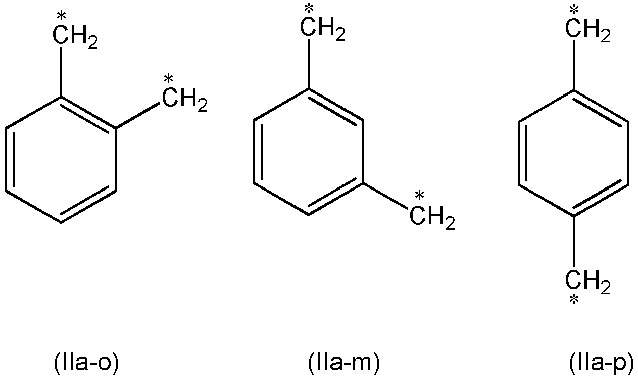 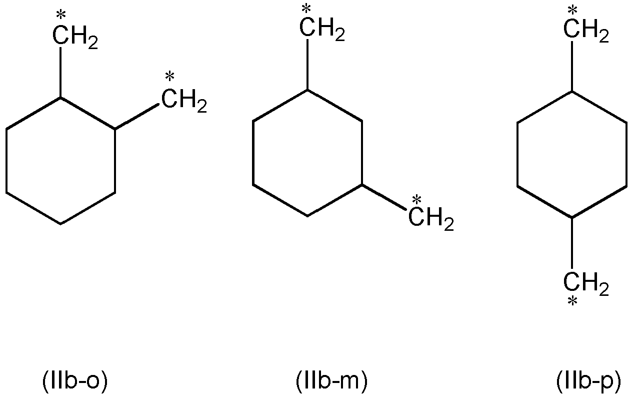 and
all n+1 R⁴ radicals are independently one or more radicals selected from the structural units (IIIa), (IIIb), (IIIc), (IIId), (IIIe), (IIIf), (IIIg) and (IIIh) 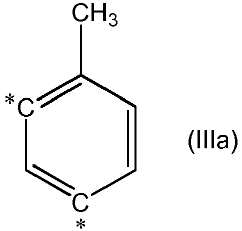 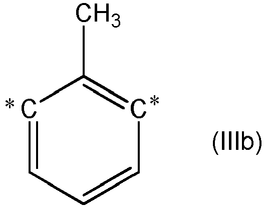 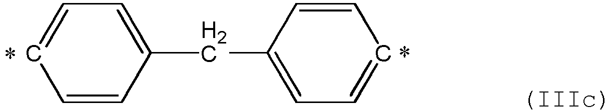 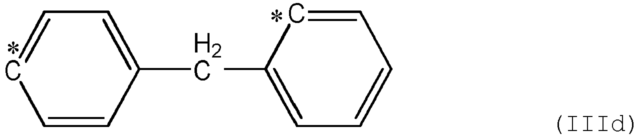 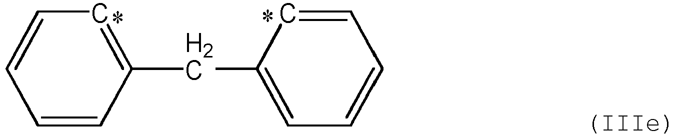 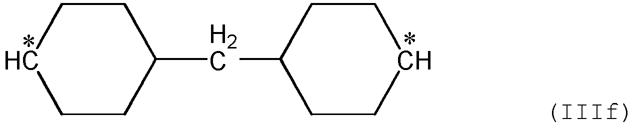 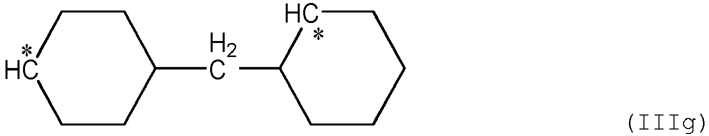 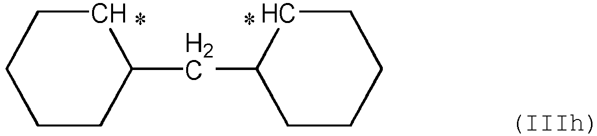 and
n is an integer ≥ 1, where the upper limit for n arises from the maximum number-average molecular weight Mₙ of the urea urethanes of the general formula (I) which is 65 000 g/mol and which is determined by means of gel permeation chromatography to DIN 55672-2 using a polymethylmethacrylate standard, and
where the bonding sites of the R³ and R⁴ radicals to the adjacent NH groups in the above formulae are indicated by the * symbol.

2. Urea urethane according to Claim 1, **characterized in that**
the R¹ radical is a monounsaturated alkenyl radical having 16 to 20 carbon atoms,
the R² radical is a saturated branched alkyl radical having 10 to 16 carbon atoms or a monounsaturated alkenyl radical having 16 to 20 carbon atoms,
the n R³ radicals are independently one or more radicals selected from the structural units (IIa-m) and (IIa-p), and
the n+1 R⁴ radicals are independently one or more radicals of the structural units (IIIa) and (IIIb).

3. Urea urethane according to either of Claims 1 and 2, **characterized in that**
the R¹ radical is an unbranched octadecenyl radical,
the R² radical is a branched or unbranched C₁₀-C₁₄-alkyl radical or an unbranched octadecenyl radical,
the n R³ radicals are a radical of the structural unit (IIa-m), and
the n+1 R⁴ radicals are independently one or more radicals of the structural units (IIIa) and (IIIb).

4. Urea urethane according to one or more of Claims 1 to 3, **characterized in that**
the structural units (IIIa) and (IIIb) are preferably present in the n+1 R⁴ radicals in a molar ratio of 40:60 up to a molar ratio of 100:0.

5. Urea urethane according to one or more of Claims 1 to 4, **characterized in that**
R¹ and R² are a mono- or polyunsaturated, branched or unbranched alkenyl radical having 16 to 20 carbon atoms.

6. Urea urethane according to one or more of Claims 1 to 5, **characterized in that** R¹ is oleyl.

7. Urea urethane according to one or more of Claims 1 to 6, **characterized in that** R¹ = R².

8. Urea urethane composition comprising one or more urea urethanes of Claims 1 to 7, **characterized in that**
the proportion by weight of the urea urethanes of the formula (I) in which both R¹ and R² radicals are unsaturated is 10% to 100%, based on the totality of the urea urethanes of the formula (I), and
the proportion by weight of the urea urethanes of the formula (I) in which only the R¹ radical is unsaturated is 0% to 90%, based on the totality of the urea urethanes of the formula (I).

9. Urea urethane composition according to Claim 8, **characterized in that** it is in liquid form.

10. Urea urethane composition according to either of Claims 8 and 9, wherein the urea urethane(s) is/are dissolved in a polar aprotic solvent.

11. Urea urethane composition according to one or more of Claims 8 to 10, wherein the polar aprotic solvent is selected from the group consisting of substituted or unsubstituted N-alkylpyrrolidones, dialkyl sulfoxides, substituted or unsubstituted amides and organic salts having a melting point of ≤ 80°C.

12. Process for preparing the urea urethane compositions according to one or more of Claims 8 to 11, **characterized in that**,
in the presence of a polar aprotic solvent,
(a) one or more monoadducts of the formula R¹-O-(CO)-NH-R⁴-NCO or of the formula R²-O-(CO)-NH-R⁴-NCO are prepared from one or more monoalcohols of the formula R¹OH and/or R²OH and one or more diisocyanates of the formula OCN-R⁴-NCO and this/these monoadduct(s) is/are then reacted
(i) with one or more diamines of the formula H₂N-R³-NH₂ or
(ii) with one or more prepolymers, NH₂-terminated at both ends, of one or more diamines of the formula H₂N-R³-NH₂ and one or more diisocyanates of the formula OCN-R⁴-NCO or
(iii) with one or more diamines of the formula H₂N-R³-NH₂ and one or more diisocyanates of the formula OCN-R⁴-NCO or
(iv) with one or more prepolymers NH₂-terminated at both ends or a mixture of one or more diamines of the structure H₂N-R³-NH₂ and one or more prepolymers NH₂-terminated at both ends, and one or more diisocyanates of the formula OCN-R⁴-NCO;
or
(b) one or more prepolymers, NCO-terminated at both ends, of one or more diisocyanates of the formula OCN-R⁴-NCO and one or more diamines of the formula H₂N-R³-NH₂ is/are prepared, and the prepolymer(s) is/are reacted with one or more monoalcohols of the formulae R¹OH and/or R²OH;
and
wherein at least one monoalcohol of the formula R¹OH is used in process variants (a) and (b).

13. Process for preparing a urea urethane (I) as defined in Claims 1 to 7, **characterized in that** the process according to Claim 12 is conducted and then the polar aprotic solvent is removed.

14. Use of the urea urethanes (I) as defined in Claims 1 to 7 or of the urea urethane compositions as defined in Claims 8 to 11 or of the urea urethane compositions obtainable by one of the processes described in Claims 12 and 13 as rheology control agents and/or antisettling agents.

15. Rheology control agent and/or antisettling agent, **characterized in that** it comprises one or more urea urethanes (I) as defined in Claims 1 to 7 or one or more urea urethane compositions as defined in Claims 8 to 11.

16. Liquid formulation selected from the group consisting of coating compositions, polymer formulations, pigment pastes, sealant formulations, cosmetics, ceramic formulations, drilling fluid solutions, nonaqueous slurries, adhesive formulations, potting compounds, building material formulations, lubricants, spackling compounds, cleaning compositions, printing inks and other inks, **characterized in that** they comprise one or more urea urethanes (I) as defined in Claims 1 to 7 or one or more of the urea urethane compositions as defined in Claims 8 to 11.

17. Liquid formulation according to Claim 16, wherein the proportion of the urea urethane (I) or of the urea urethanes (I) in the overall liquid formulation is 0.1% to 5% by weight.

18. Liquid formulation according to either of Claims 16 and 17, **characterized in that** at least one hydrocarbon selected from the group of the aliphatic, cycloaliphatic, aromatic and araliphatic hydrocarbons is additionally present in the liquid formulation.

19. Liquid formulation according to Claim 18, wherein the hydrocarbon(s) is/are present in the liquid formulation to an extent of at least 10% by weight based on the total weight of the liquid formulation.

## Revendications

1. Urées-uréthanes de formule générale (I) 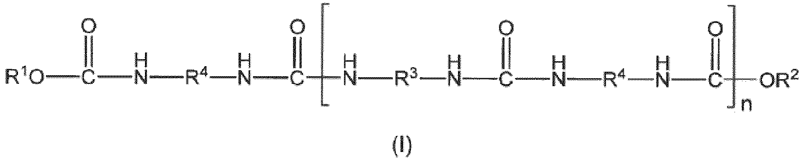 dans laquelle
R¹ représente un radical alcényle mono- ou polyinsaturé, ramifié ou non ramifié, de 12 à 24 atomes de carbone, un radical alcynyle mono- ou polyinsaturé, ramifié ou non ramifié, de 12 à 24 atomes de carbone, ou un radical hydrocarboné polyinsaturé de 12 à 24 atomes de carbone, qui comprend au moins une double liaison carbone-carbone et au moins une triple liaison carbone-carbone,
R² représente un radical alkyle saturé, ramifié ou non ramifié, de 8 à 24 atomes de carbone, ou un radical alcényle mono- ou polyinsaturé, ramifié ou non ramifié, de 12 à 24 atomes de carbone, un radical alcynyle mono- ou polyinsaturé, ramifié ou non ramifié, de 12 à 24 atomes de carbone, ou un radical hydrocarboné polyinsaturé de 12 à 24 atomes de carbone, qui comprend au moins une double liaison carbone-carbone et au moins une triple liaison carbone-carbone, et
les n radicaux R³ représentent tous indépendamment les uns des autres un ou plusieurs radicaux choisis parmi les unités structurales (IIa-o), (IIa-m), (IIa-p), (IIb-o), (IIb-m) et (IIb-p) 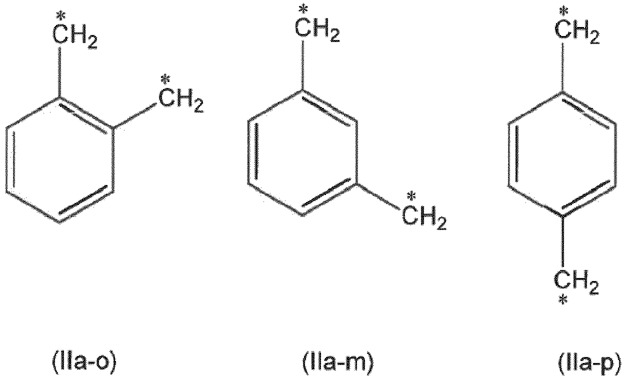 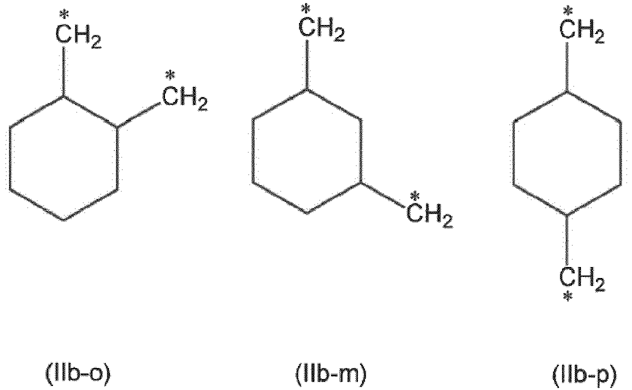 et
les n+1 radicaux R⁴ représentent tous indépendamment les uns des autres un ou plusieurs radicaux choisis parmi les unités structurales (IIIa), (IIIb), (IIIc), (IIId), (IIIe), (IIIf), (IIIg) et (IIIh) 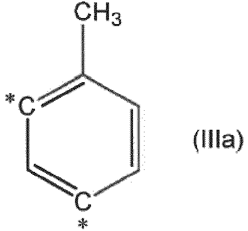 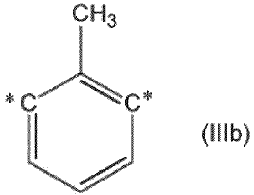 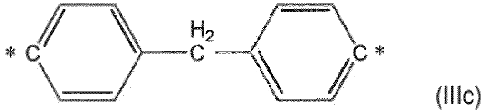 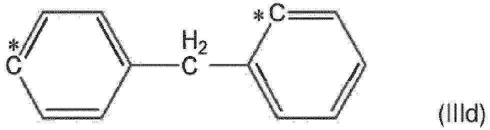 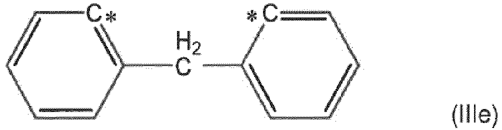 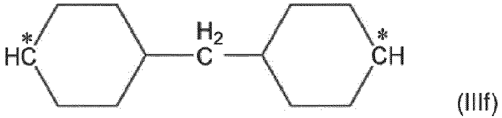 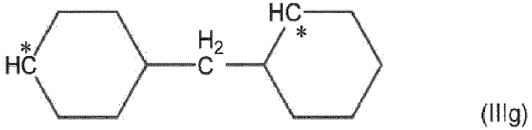 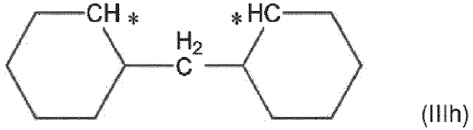 et
n représente un nombre entier ≥ 1, la limite supérieure pour n étant donnée par le poids moléculaire moyen en nombre maximal Mₙ des urées-uréthanes de formule générale (I), qui est de 65 000 g/mol et qui est déterminé par chromatographie par perméation de gel selon DIN 55672-2 en utilisant un étalon polyméthacrylate de méthyle,
les emplacements de liaison des radicaux R³ et R⁴ aux groupes NH voisins dans les formules ci-dessus étant désignés par le symbole étoile *.

2. Urées-uréthanes selon la revendication 1, **caractérisées en ce que**
le radical R¹ représente un radical alcényle monoinsaturé de 16 à 20 atomes de carbone,
le radical R² représente un radical alkyle saturé ramifié de 10 à 16 atomes de carbone, ou un radical alcényle monoinsaturé de 16 à 20 atomes de carbone,
les n radicaux R³ représentent indépendamment les uns des autres un ou plusieurs radicaux choisis parmi les unités structurales (IIa-m) et (IIa-p), et
les n+1 radicaux R⁴ représentent indépendamment les uns des autres un ou plusieurs radicaux des unités structurales (IIIa) et (IIIb).

3. Urées-uréthanes selon l'une quelconque des revendications 1 ou 2, **caractérisées en ce que**
le radical R¹ représente un radical octadécényle non ramifié,
le radical R² représente un radical alkyle en C₁₀-C₁₄ ramifié ou non ramifié, ou un radical octadécényle non ramifié,
le n radicaux R³ représentent un radical de l'unité structurale (IIa-m), et
les n+1 radicaux R⁴ représentent indépendamment les uns des autres un ou plusieurs radicaux des unités structurales (IIIa) et (IIIb).

4. Urées-uréthanes selon une ou plusieurs des revendications 1 à 3, **caractérisées en ce que**
dans les n+1 radicaux R⁴, les unités structurales (IIIa) et (IIIb) sont de préférence présentes en un rapport molaire de 40 sur 60 à un rapport molaire de 100 sur 0.

5. Urées-uréthanes selon une ou plusieurs des revendications 1 à 4, **caractérisées en ce que**
R¹ et R² représentent un radical alcényle mono- ou polyinsaturé, ramifié ou non ramifié, de 16 à 20 atomes de carbone.

6. Urées-uréthanes selon une ou plusieurs des revendications 1 à 5, **caractérisées en ce que** R¹ représente oléyle.

7. Urées-uréthanes selon une ou plusieurs des revendications 1 à 6, **caractérisées en ce que** R¹ = R².

8. Compositions d'urée-uréthane, contenant un ou plusieurs des urées-uréthanes selon les revendications 1 à 7, **caractérisées en ce que**
la proportion en poids des urées-uréthanes de formule (I) dans lesquelles les deux radicaux R¹ et R² sont insaturés est de 10 % à 100 %, par rapport à la totalité des urées-uréthanes de formule (I), et
la proportion en poids des urées-uréthanes de formule (I) dans lesquelles uniquement le radical R¹ est insaturé est de 0 % à 90 %, par rapport à la totalité des urées-uréthanes de formule (I).

9. Composition d'urée-uréthane selon la revendication 8, **caractérisée en ce que** celle-ci est liquide.

10. Composition d'urée-uréthane selon l'une quelconque des revendications 8 ou 9, dans laquelle la ou les urées-uréthanes se présentent dissous dans un solvant aprotique polaire.

11. Composition d'urée-uréthane selon une ou plusieurs des revendications 8 à 10, dans laquelle le solvant aprotique polaire est choisi dans le groupe constitué par les N-alkylpyrrolidones substituées ou non substituées, les dialkylsulfoxydes, les amides substitués ou non substitués, et les sels organiques ayant un point de fusion ≤ 80 °C.

12. Procédé de fabrication des compositions d'urée-uréthane selon une ou plusieurs des revendications 8 à 11, **caractérisé en ce que**
en présence d'un solvant aprotique polaire,
(a) un ou plusieurs monoadduits de formule R¹-O-(CO)-NH-R⁴-NCO ou de formule R²-O-(CO)-NH-R⁴-NCO d'un ou de plusieurs monoalcools de formule R¹OH et/ou R²OH et d'un ou de plusieurs diisocyanates de formule OCN-R⁴-NCO sont fabriqués, et ce monoadduit ou ces monoadduits sont ensuite mis en réaction
(i) avec une ou plusieurs diamines de formule H₂N-R³-NH₂ ou
(ii) avec un ou plusieurs prépolymères à terminaison NH₂ des deux côtés d'une ou de plusieurs diamines de formule H₂N-R³-NH₂ et d'un ou de plusieurs diisocyanates de formule OCN-R⁴-NCO, ou
(iii) avec une ou plusieurs diamines de formule H₂N-R³-NH₂, ainsi qu'un ou plusieurs diisocyanates de formule OCN-R⁴-NCO, ou
(iv) avec un ou plusieurs prépolymères à terminaison NH₂ des deux côtés ou un mélange d'une ou de plusieurs diamines de structure H₂N-R³-NH₂ et d'un ou de plusieurs prépolymères à terminaison NH₂ des deux côtés, ainsi qu'un ou plusieurs diisocyanates de formule OCN-R⁴⁻NCO ; ou
(b) un ou plusieurs prépolymères à terminaison NCO des deux côtés d'un ou de plusieurs diisocyanates de formule OCN-R⁴-NCO et d'une ou de plusieurs diamines de structure H₂N-R³-NH₂ est ou sont fabriqués, et le ou les prépolymères est ou sont mis en réaction avec un ou plusieurs monoalcools des formules R¹OH et/ou R²OH ;
au moins un monoalcool de formule R¹OH étant utilisé dans les variantes de procédé (a) et (b).

13. Procédé de fabrication d'une urée-uréthane (I) telle que définie dans les revendications 1 à 7, **caractérisé en ce que** le procédé selon la revendication 12 est réalisé, puis le solvant aprotique polaire est éliminé.

14. Utilisation des urées-uréthanes (I) telles que définies dans les revendications 1 à 7 ou des compositions d'urée-uréthane telles que définies dans les revendications 8 à 11 ou des compositions d'urée-uréthane pouvant être obtenues par un des procédés décrits dans les revendications 12 et 13 en tant qu'agent d'ajustement de la rhéologie et/ou agent stabilisateur.

15. Agent d'ajustement de la rhéologie et/ou agent stabilisateur, **caractérisé en ce que** celui-ci contient un ou plusieurs urées-uréthanes (I) telles que définies dans les revendications 1 à 7 ou une ou plusieurs des compositions d'urée-uréthane telles que définies dans les revendications 8 à 11.

16. Formulation liquide choisie dans le groupe constitué par les agents de revêtement, les formulations de matière plastique, les pâtes de pigments, les formulations d'agents d'étanchéité, les cosmétiques, les formulations de céramiques, les fluides de forage, les suspensions non aqueuses, les formulations d'adhésifs, les masses de scellement, les formulations de matériaux de construction, les lubrifiants, les mastics, les détergents, les encres d'impression et les encres, **caractérisée en ce que** celle-ci contient une ou plusieurs urées-uréthanes (I) telles que définies dans les revendications 1 à 7 ou une ou plusieurs des compositions d'urée-uréthane telles que définies dans les revendications 8 à 11.

17. Formulation liquide selon la revendication 16, dans laquelle la proportion de l'urée-uréthane (I) ou des urées-uréthanes (I) dans la formulation liquide totale est de 0,1 à 5 % en poids.

18. Formulation liqide selon l'une quelconque des revendications 16 ou 17, **caractérisée en ce qu'**au moins un hydrocarbure choisi dans le groupe des hydrocarbures aliphatiques, cycloaliphatiques, aromatiques et araliphatiques est en outre contenu dans la formulation liquide.

19. Formulation liquide selon la revendication 18, dans laquelle l'hydrocarbure ou les hydrocarbures est ou sont contenus dans la formulation liquide à hauteur d'au moins 10 % en poids, par rapport au poids total de la formulation liquide.
